# EUROPEAN PATENT SPECIFICATION

(11) **EP 2 723 333 B1**
(45) Date of publication and mention of the grant of the patent: **16.09.2015**
(21) Application number: 12744116.0
(22) Date of filing: 25.06.2012
(51) Int. Cl.: A61K 31/198, A61P 5/50, A61P 5/00, A61P 7/06, A61P 9/10, A61P 13/12, A61P 35/00, A61P 3/10, A61P 3/04, A23L 1/305, A61K 31/192

(54) **TREATMENT AND PREVENTION OF DISEASES RELATED TO OXIDATIVE STRESS**
BEHANDLUNG UND PRÄVENTION VON ERKRANKUNGEN IN BEZIEHUNG MIT OXIDATIVER BELASTUNG
TRAITEMENT ET PRÉVENTION DES MALADIES LIÉES AU STRESS OXYDATIF

(30) Priority: 23.06.2011 US 201161500153 P; 06.12.2011 EP 11462024
(43) Date of publication of application: 30.04.2014
(73) Proprietor: University Of Pécs, 7622 Pécs (HU)
(72) Inventor: WITTMANN, István, H-7631 Pécs (HU); MOLNÁR, Gergo Attila, H-7629 Pécs (HU); MOHAS-CSEH, Judit, H-7634 Pécs (HU); SZIJÁRTÓ, István András, H-8330 Sümeg (HU)
(74) Representative: Svingor, Adam
(86) International application number: PCT/HU2012/000055
(87) International publication number: WO 2012/176009

(56) References cited:
- WO-A2-01/13935
- US-A1- 2008 090 832
- US-B1- 7 977 319

## Description

### Field of the Invention

The present invention relates to the field of treatment and prevention of conditions due to oxidative stress and hormone resistance. Preferably, insulin resistance, erythropoietin resistance and acetyl-choline resistance are in the focus. The invention also relates to the field of use of amino acids, in particular p-L-Tyrosine and the precursor thereof as a medicament or composition or formulation in the prevention or treatment of said conditions.

### Background Art

Adipose tissue is an active endocrine and paracrine organ that influences not only body weight homeostasis but also plays an important role in inflammation, which induces oxidative stress (OS) leading to e.g. insulin resistance and diabetes mellitus (Iozzo 2009, Houstis 2006). OS leads to an imbalance between reactive oxygen species (ROS) and antioxidant defence capacity. ROS involve among others superoxide anion radical (-O₂⁻), hydrogen peroxide (H₂O₂) and hydroxyl free radical (·OH). The -OH may be formed e.g. in Fenton reaction in the presence of transition metals. Oxidative stress (OS) is known to have a negative effect on macromolecules; for example OH is able to convert L-phenylalanine (Phe) into *meta*-tyrosine (m-Tyr) and *ortho*-tyrosine (o-Tyr) (Huggins 1993), isomers of the natural amino acid *para*-tyrosine (p-Tyr). This reaction can take place on Phe residues inside protein polypeptide chains, but also on non-protein-bound amino acids (Galano2008). Because m-Tyr and o-Tyr are stable molecules and thought to be absent from normal proteins, they have served as useful markers of -OH radical stress (Gurer-Orhan 2006).

OS related diseases are usually suggested to be treated and/or prevented with direct antioxidants, like ascorbic acid, tocopherols, carotenoids and polyphenols (Pokomy 2001). A therapeutic intervention by the administration of the antioxidant species resveratrol might lead to a decrease in insulin resistance (HOMA_{IR}) (Brasnyó 2011). Alternatively agents that increase the levels of intracellular antioxidants, like glutathione (US2009/0017002A1) are administered. Typically, these efforts were mainly ineffective and most clinical studies did not confirm previous assumptions. However, previous data of our workgroup showed that, resveratrol is able to improve insulin resistance, by decreasing the urinary excretion of o-Tyr (Brasny6 2011).

The present inventors are not aware of any prior art information on using para-L-Tyrosine (also used herein as para-Tyrosine, p-Tyr or Tyr in short), its derivatives, precursors or related compounds in the treatment of diseases due to or induced by OS.

US2010/0172916A1 provides a review on medical uses of meta- or para-Tyrosine and meta or para-Tyramine. Tyrosine, being a neurotransmitter precursor, is widely used as a dietary supplement.

US 7977319 teaches a high fiber content drink which may comprise, among other amino acids, p-Tyr as well. There is no indication of the fact that p-Tyr itself, as an active agent, would be appropriate to prevent a hormone resistance disease.

In WO 01/13935 the treatment of leptin resistance is suggested by using, among others, p-Tyr, by increasing the leptin transport across the blood-brain barrier and thereby treating obesity.

US 2008/0090832 teaches the treatment of hyperphenylalaninemia by administering tetrahydrobiopterin to a subject. p-Tyr is mentioned as an amino acid dietary supplement only and not as an active agent.

Reinstein et al. (Reinstein 1985) propose the use of dietary tyrosine in conditions with reductions in stress hormone levels.

Hao et al. suggest that tyrosine might be a potential therapy for cognitive and mood problems associated with the maintenance of a reduced body weight in the treatment of obesity and in the extreme case of anorexia nervosa (Hao 2001). In this regard, it is reported that tyrosine may reduce appetite in susceptible subjects (Møller 1995), e.g. by affecting the hypothalamus, a brain area involved in appetite regulation (Fernstrom 2001).

The present inventors have unexpectedly found that while m-Tyr and o-Tyr, formed under oxidative stress conditions, inhibit protein phosphorylation in the P13-K/Akt (phosphatidyl-inositol-3 kinase/protein kinase B) pathway, p-Tyr can reverse this process.

The present inventors have also found that p-Tyr is useful to reverse hormone resistance developed due to formation of ortho-Tyr and/or m-Tyr in the cells due to OS and incorporation of these amino acids into receptor proteins of said hormone or signalling proteins of the respective signal transduction pathway. In preferred embodiments, p-Tyr is useful to reverse insulin resistance and/or erythropoietin (EPO) resistance and/or acetylcholine resistance.

Moreover, the present inventors have recognized that p-Tyr is useful in the treatment of hormone resistance diseases mediated or induced by oxidative stress, said hormone resistance diseasebeing selected from the group of diseases, conditions or syndromesobesity, IGT (impaired glucose tolerance), metabolic syndrome, type 2 diabetes mellitus, hypertension, insulin resistance; oxidative stress in adipose tissue/fat cells, anemia, chronic anemia, ischemia, vascular diseases, chronic kidney disease (CKD), chronic heart failure (CHF), oxidative stress of the kidney, obstructive sleep apnea (OSAS) (or other diseases related to the insulin or erythropoietin or acetyl-choline (signaling) pathway).

### Brief Description of the Invention

The present invention relates to a compound selected from para-L-Tyrosine (p-Tyr) and p-OH-phenyl-pyruvate or pharmaceutically acceptable salts thereof, for use in a subject in the prevention, amelioration or treatment of a condition due to hormone resistance (or hormone resistance disease) induced by oxidative stress, wherein said p-OH-phenyl pyruvate is metabolized to p-Tyr in said subject.

In a preferred embodiment the subject shows or is characterized by a state of oxidative stress, preferably chronic oxidative stress, preferably a hydroxyl radical damage on phenylalanine (Phe). Preferably, at least one oxidative stress marker is increased in said patient, preferably in a biological sample obtained from said patient. Optionally the urinary level of m-Tyr and o-Tyr can be regarded as specific markers of hydroxyl radical damage on Phe. Furthermore, a high-normal or increased level of high sensitivity C-reactive protein (hsCRP) in a body fluid of said patient, wherein the body fluid is serum or plasma, may be regarded as an indirect marker of oxidative stress.

In a preferred embodiment, said hormone resistance is insulin resistance.

In a preferred embodiment, said hormone resistance is erythropoietin resistance.

In a further preferred embodiment said hormone resistance is acetyl-choline resistance.

Preferably, said condition is selected from obesity, IGT (impaired glucose tolerance), metabolic syndrome, type 2 diabetes mellitus, hypertension, insulin resistance, erythropoietin resistance, acetyl-choline resistance, oxidative stress in adipose tissue/fat cells, anemia, chronic anemia, ischemia, vascular diseases, chronic kidney disease (CKD), chronic heart failure (CHF), oxidative stress of the kidney, obstructive sleep apnea (OSAS) or other diseases due to the insulin or erythropoietin or acetyl-choline (signaling) pathway).

Highly preferably the condition is selected from hypertension, anemia, e.g. chronic anemia, vascular diseases, chronic heart failure (CHF), even more preferably the condition is hypertension or anemia, Highly preferably, the condition is a kidney disease, e.g. chronic kidney disease (CKD) or oxidative stress of the kidney.

In a preferred embodiment the hormone resistance is induced by oxidative stress via the formation of o-Tyro and/or m-Tyr and optionally via incorporation of said o-Tyr and/or m-Tyr into cellular proteins, preferably into at least one cellular protein of the intracellular signalling pathway of a hormone the resistance of which said condition is related to.

Preferably, the hormone resistance is different from leptin resistance. In a further preferred embodiment insulin resistant pathologies which are due to an inhibition or blockade of the transport of leptin across the blood-brain barrier are excluded from the scope of the present invention. In particular, in a preferred embodiment insulin resistance and/or type 2 diabetes mellitus and/or obesity due to, induced by, or mediated by leptin resistance is excluded. This scope of solutions can be assessed or defined by measuring leptin level in sera of said patients. Typically, leptin resistant patients have an elevated serum leptin level.

In a further preferred embodiment compositions and dietary regimes wherein p-Tyr is used as an amino acid dietary supplement and not as an active agent are excluded from the scope of the present invention.

Preferably, the hormone resistance is induced by oxidative stress via modifying tyrosine residues of at least one cellular protein of the intracellular signalling pathway of a hormone the resistance of which said condition is related to, thereby inhibiting tyrosine phosphorylation of or by said protein.

More preferably, said at least one protein is selected from proteins of the glucose uptake and/or vasodilation pathway of insulin signaling pathway; and/or proteins of the neuroprotective, phosphatidylinositol neuroprotective and/or erythrocyte generating pathway of erythropoietin signalling pathway; and/or proteins of the phosphatidylinositol 3-kinase(PI3K)/AKT pathway. More preferably, said at least one protein is selected from the intracellular part of a receptor of said hormone, insulin receptor, IRS1, IRS2, PD3K, PDKI, AKT and GLUT4.

In a preferred embodiment the hormone acts via the phosphatidylinositol 3-kinase(PI3K)/AKT pathway.

In a further embodiment the prevention, amelioration or treatment of said condition comprises, consisting of or being a part of a (personalized) regimen or administration schedule tailored to said subject.

In a preferred embodiment, the patient to be treated according to the invention is a patient exposed to oxidative stress or a patient being in the state of oxidative stress. Preferably oxidative stress is chronic oxidative stress. A patient exposed to oxidative stress, preferably chronic oxidative stress, preferably due to an oxidative stress disease has developed or is at risk of developing a condition or disorder due to hormone resistance induced by oxidative stress.

Thus, the invention preferably applicable when a condition or disorder due to hormone resistance induced by oxidative stress is to be prevented or ameliorated, preferably prevented.

The state of oxidative stress can be shown or diagnosed via oxidative stress markers.

In a preferred embodiment the oxidative stress marker is elevated level or excretion, e.g. daily excretion of m-Tyr or o-Tyr in the urine of the patient A further oxidative stress marker is the high-normal or elevated level of high sensitivity C-reactive protein (hsCRP).

Thus, in a further embodiment the present invention relates to a compound selected from para-L-Tyrosine (p-Tyr), p-Tyr precursor or pharmaceutically acceptable salts thereof, wherein said p-Tyr precursor is metabolized to p-Tyr in said subject, for use in a subject in the prevention or amelioration, or preferably prevention of a condition due to hormone resistance wherein said subject is exposed to oxidative stress and/or oxidative stress due to hydroxyl free radical (-OH) formation or said subject suffers in an oxidative stress disease. Preferably oxidative stress is chronic oxidative stress and/or oxidative stress disease is a chronic oxidative stress disease.

In a preferred embodiment the subject has a subclinical inflammation condition. In particular, the subject is characterized by a high-normal or elevated level of high sensitivity C-reactive protein (hsCRP).

In a further preferred embodiment the patient shows an elevated level or excretion, e.g. daily excretion of m-Tyr or o-Tyr in the urine.

Preferably said subject is a mammal, preferably a human.

Said compound is selected from
- para-L-Tyrosine and a p-OH-phenyl-pyruvate.

In a preferred embodiment said compound is formulated as a pharmaceutical composition or a medicament, or the compound is formulated as a dietary supplement, nutraceutical, functional food, food or composition with a health claim.

The invention also relates to said formulation, preferably selected from pharmaceutical compositions, dietary supplements, nutraceuticals, functional foods, foods or compositions with a health claim. Preferably said formulation is for use in the treatment of a condition as defined above. Preferably, said formulation is accompanied by a description of biological effect of the compound of the invention and/or the therapeutic indication and/or the proposed treatment each of them as disclosed herein.

The formulation may be formulated for oral use or parenteral use. In an embodiment, the formulation is formulated as a pharmaceutical composition or a medicament and further comprising a pharmaceutically acceptable carrier or excipient.

In a further embodiment, the formulation is formulated as a dietary supplement, food supplement, nutraceutical, functional food, food or composition, or one or more of them with a health claim.

In a preferred embodiment the formulation of the invention comprises at least one dose or multiple doses of the compound suitable to provide daily intake for at least one day.

In an embodiment a single dose of said compound is at least 100 mg, 200 mg, 300, mg, 400 mg, 500 mg, 600 mg, 700 mg, 800 mg, 900 mg, 1 g and/or at most 10 g, 7.5 g, 5 mg, 3 g, 2.5 g, 2 g, 1.8 g, 1.6 g, 1.5 g, 1.4 g, 1.3 g, 1.2 g, 1.1 g, 1 g, 900 mg, 800 mg, 700 mg, 600 mg, 500 mg, depending on the maximum or minimum dose, respectively.

The invention further provides a method for preventing, ameliorating or treating a condition related to hormone resistance induced by oxidative stress, comprising administering a compound or a pharmaceutically effective dose of a compound selected from p-Tyr, p-Tyr precursor or pharmaceutically acceptable salts thereof, wherein said p-Tyr precursor is metabolized to p-Tyr in said subject.

Preferably, the condition is a condition as defined above.

Preferably, in the method of the disclosure the compound administered is a compound as defined above.

Preferably, the subject having said condition to be prevented, ameliorated or treated is a mammal, preferably a human.

Preferably, the prevention, amelioration or treatment of said condition comprises, consisting of or being a part of a (personalized) regimen or administration schedule tailored to said subject.

In a preferred option the compound is administered to a subject exposed to oxidative stress and/or oxidative stress due to hydroxyl free radical (-OH) formation or a subject in an oxidative stress disease. Preferably oxidative stress is chronic oxidative stress and/or oxidative stress disease is a chronic oxidative stress disease. In a preferred option the subject has a subclinical inflammation condition. In particular, the subject is characterized by a high-normal or elevated level of high sensitivity C-reactive protein (hsCRP). In a further preferred option the patient shows an elevated level or excretion, e.g. daily excretion of m-Tyr or o-Tyr in the urine.

In a preferred option the daily intake of said compound is one, two or three times a day of a dose of at least 100 mg, 200 mg, 300, mg, 400 mg, 500 mg, 600 mg, 700 mg, 800 mg, 900 mg, 1 g and/or at most 10 g, 7.5 g, 5 mg, 3 g, 2.5 g, 2 g, 1.8 g, 1.6 g, 1.5 g, 1.4 g, 1.3 g, 1.2 g, 1.1 g, 1 g, 900 mg, 800 mg, 700 mg, 600 mg, 500 mg. If appropriate, single larger doses may be applied for a single daily intake of up to 30 000 mg provided that it can be formulated in a single dosage unit which can still be orally administered, e.g. swallowed. and is administered in. Preferably, the formulation of the invention is formulated in a form suitable for administration of a dose providing the daily intake.

In a further preferred option serum level of p-Tyr and o-Tyr and m-Tyr is monitored during setting the p-Tyr dosage regime or during treatment.

In a preferred option monitoring includes measurement of serum and urinary p-Tyr and o-Tyr and/or m-Tyr levels as well as, preferably, serum glucose and insulin levels. In a certain option also the uptake of p-Tyr and/or the hormone by adipocytes and/or adipose tissue is monitored.

In a preferred option of the treatment for a condition or disease due to hormone resistance induced by oxidative stress
- p-Tyr or a p-Tyr precursor is administered to a subject characterized by or predisposed to a condition or disorder due to hormone resistance induced by oxidative stress,
- the oxidative stress level is assessed in said patient,
- wherein if the oxidative stress level is above a predetermined value, the daily dose of p-Tyr or a p-Tyr precursor administered is increased in said patient.

Preferably, the administration is carried out in a continuous treatment or is continued for at least 7, 8, 9, 10, 11, 12, 13, 14 or 15 days, more preferably for at least one month, at least 2 months, at least 3 months, at least 4 months or at least for half year or at least 1 year or at least 2 years or at least 3 years or at least 5 years or at least 10 years or life long.

Said compound is preferably formulated defined as above.

The invention also provides a use of a compound according to the invention for the preparation of a formulation as defined herein for use in the prevention, amelioration or treatment of a condition as defined herein.

### Definitions

*Para-Tyrosine precursors* are molecules which are converted to para-L-Tyrosine in the living body of an animal, including humans. Preferably, para-L-Tyrosine precursors are synthesized in the living body of said animal. Highly preferably, according to the invention the para-Tyrosine precursor is p-OH-phenyl-pyruvate, the ketoanalogue of p-Tyr.

A *cellular protein* is a protein, when expressed by a cell, is located within said cell or the cellular side of the membrane of said cell, preferably within said cell.

*Signalling pathway* or *signal transduction pathway* or simply pathway, if context allows, is used herein interchangeably and relates to a series of biochemical changes and or events including biochemical changes and/or events within the cell wherein said changes or events are influenced, catalysed or effected by biochemical substances e.g. biomolecules and wherein a cell membrane receptor receives a signal (preferably a hormone) and transmits this information into the cell and, preferably effects a cellular response, or relates to a part of this process, preferably comprising at least two biochemical changes events or reactions preferably involving at least two enzymes.

Receptors that initiate biochemical changes can do so either directly via intrinsic enzymatic activities within the receptor or by activating intracellular messenger molecules.

A *hormone* is a chemical substance produced in the body that controls and regulates the activity of certain cells or organs. Thus this term is understood broadly herein including small chemical molecules that carry messages in the body, e.g. from organs to cells, thus including e.g. acetyl-choline. In a further preferred embodiment a hormone is a peptide hormone, e.g. insulin or erythropoietin. Preferably, the hormone is different from a leptine.

A *condition or disease due to hormone resistance (or a hormone resistance disease)* is to be understood herein as the status of a patient wherein hormone resistance in said patient contributed to the status. Said status is different from a healthy status, e.g. is characterized by measurable parameters which fell outside the range accepted as normal or typical of a healthy subject. Optionally, said status is a disease e.g. a disease which is due to hormone resistance formed in said patient.

The term *hormone resistance induced by oxidative stress* (or a *hormone resistance disease mediated by oxidative stress)* relates to a condition wherein oxidative stress is detectably present in the body of a patient. If oxidative stress is shown by the elevated level of an oxidative stress marker as defined herein it presents an increased risk of hormone resistance, preferably insulin resistance and/or erythropoietin resistance and/or acetylcholine resistance. Thus, the state of oxidative stress, preferably chronic oxidative stress defines a group of subjects to be treated in accordance with the present invention. Thereby *a condition or disease due to hormone resistance. induced by oxidative stress or an oxidative stress diseases* can be prevented or ameliorated. In practice, the term *hormone resistance induced by oxidative stress or an oxidative stress disease* is to be understood that there is a co-existence or correlation between hormone resistance and oxidative stress in the given subject or patient.

*Oxidative stress* (OS) is the state of a subject characterized by an imbalance between reactive oxygen species (ROS) and antioxidant defence capacity or the subject, i.e. an increased level of ROS, in particular superoxide anion radical (·O₂⁻), hydrogen peroxide (H₂O₂) and hydroxyl free radical (-OH) in a highly preferred embodiment -OH. Oxidative stress, if persistent, is chronic oxidative stress. Chronic oxidative stress is understood herein as the presence of oxidative stress in the subject for a time period longer than in acute oxidative stress, i.e. for typically at least 5 days or at least 10 days or at least 15 days or for 1 week or for at least 2 weeks or for at least 3 weeks or, preferably, for at least 4 weeks or for at least 1 month or at least 2 months or at least 3 months or for at least for a half year or for at least a year.

Oxidative stress disease or chronic oxidative stress disease is a condition of a subject characterized by a chronic oxidative stress wherein in said subject any harmful effect or symptom of a chronic oxidative stress, e.g. the occurrence of hormone resistance, preferably insulin resistance, and/or EPO resistance and/or acetyl-choline resistance can be shown, assessed, measured or diagnosed. Methods for assessment of oxidative stress are disclosed herein as well as known in the art.

A *subject* is a human being or an animal. A *patient* is a subject who receives medical attention, care, or treatment.

*Prevention* as understood herein relates to the treatment of a subject comprising administration of the compound of the invention with the aim of preventing the development of hormone resistance in said subject or preventing onset of a hormone resistance disease or preventing exacerbation of the symptoms of a hormone resistance disease. Preferably, *prevention* is a treatment of a subject exposed to, characterized by or having the status of oxidative stress, preferably chronic oxidative stress in order to prevent the development of hormone resistance in said subject or prevent onset of a hormone resistance disease. Partial prevention is preferably included in the meaning of this term.

A *biochemical process* comprises one or more functionally related biological changes and/or events in a living organism, having an effect in said organism, said effect being preferably detectable, wherein said changes or events are influenced, catalysed or effected by biochemical substances e.g. biomolecules.

*Inhibition* of a biochemical process is meant as a process resulting in reduced biological effect of said biological process in comparison with the same process without inhibition. Said reduction of biological effect may result in the abolishment of said effect, may result in the reduction of said effect to a level which is not detectable or the reduction of said effect to a level lower than that measured without inhibition.

*Inhibitor* is a substance, preferably a compound contacting of which with said biochemical substances e.g. biomolecules results in inhibition.

**Non-standard Abbreviations and Acronyms**

| | |
|---|---|
| ACh | Acetylcholine |
| ERK | Extracellular signal-regulated kinase |
| HPLC | High-performance liquid chromatography |
| m-Tyr | Meta-L-tyrosine |
| NO | Nitric oxide |
| o-Tyr | Ortho-L-tyrosine |
| -OH | Hydroxyl radical |
| p-Tyr | Para-L-tyrosine |
| Phe | L-phenylalanine |
| PI3K/Akt | Phosphatidylinositol 3-kinase/Akt |
| Px | Peroxidases |
| ROS | Reactive oxygen species |

### Brief Description of the Figures

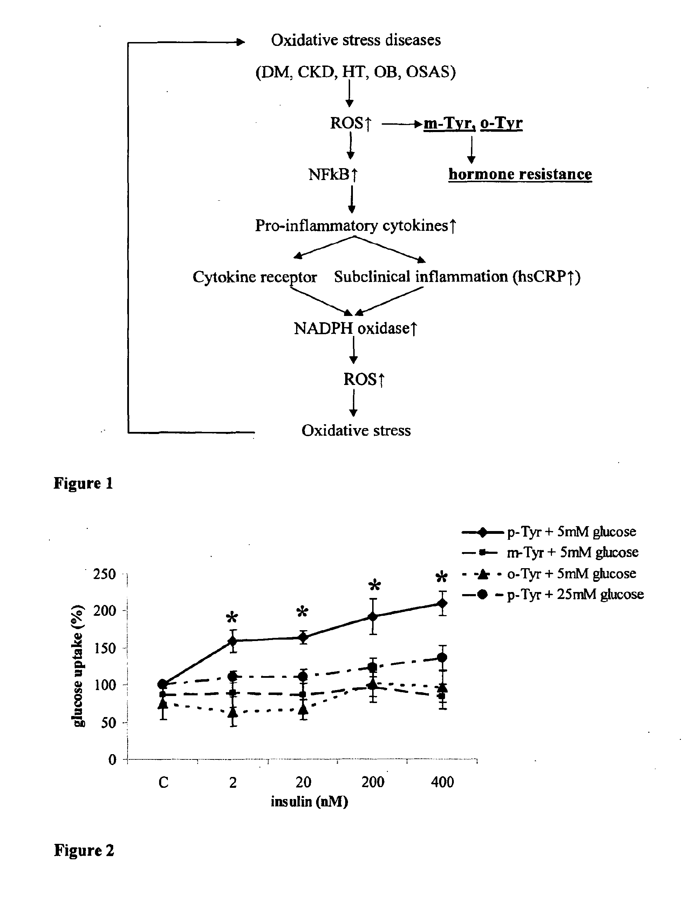

**Figure 1****.** A schematic presentation of the relationship between oxidative stress, meta- and ortho-tyrosine production, subclinical inflammation and a consequent hormone resistance. Persistent and untreated oxidative stress through a feed-forward (or positive feed-back) mechanism initiates a vicious circle leading to hormone resistance and oxidative stress diseases.
**Figure 2****.** Insulin-dependent uptake of deoxy-D-glucose 2-[1 2-³H(N)] into 3T3-L1 adipocytes. Glucose uptake was measured at media containing i) para-tyrosine with 5 mM glucose content, ii) meta-tyrosin with 5 mM glucose, iii) ortho-tyrosine with 5 mM glucose and iv) para-tyrosine with 25 mM glucose content. Cells were treated with 2, 20, 200 and 400 nM insulin as shown. The glucose uptake of untreated cells grown on para-tyrosine and 5 mM glucose medium was set to 100%. Results are mean ± SEM for n=5 individual measurements. *,p<0.05 p-Tyr + 5 mM glucose vs. m-Tyr + 5 mM glucose, o-Tyr + 5 mM glucose and p-Tyr + 25 mM glucose, at all insulin concentrations (independent samples t-test).

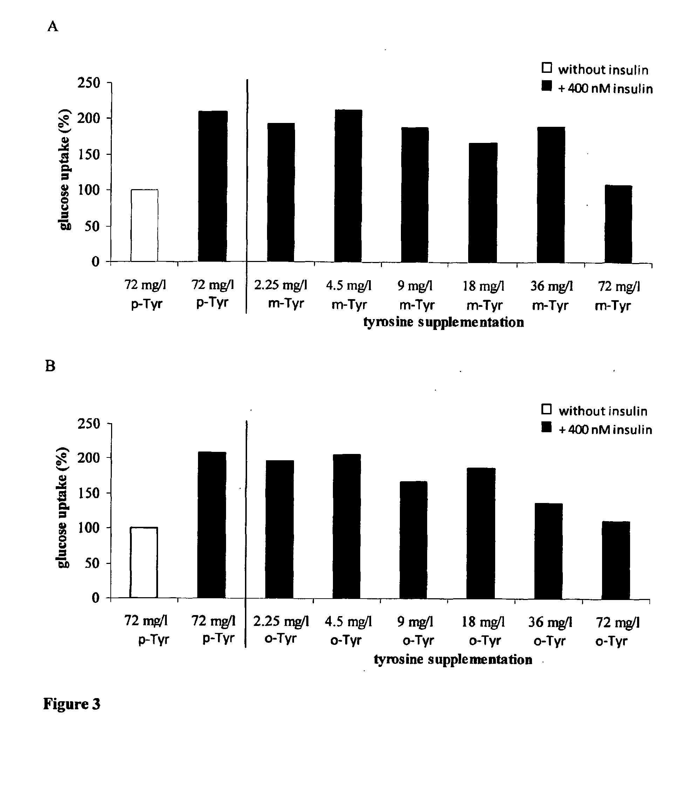

**Figure 3****.** Insulin-dependent (400 nmol/l) uptake of deoxy-D-glucose 2-[1 2-3H(N)] into 3T3-L1 adipocytes (filled charts) in media, supplemented with para tyrosine (72 mg/l) or either meta (panel A) or ortho (panel B) tyrosine in different concentrations (72 mg/l, 36 mg/l, 18 mg/l, 9 mg/l, 4.5 mg/l, 2.25 mg/l). Open bars indicate the uptake of the insulin-untreated (control) cells grown on para-tyrosine medium. Results are representative for n=2 individual measurements.

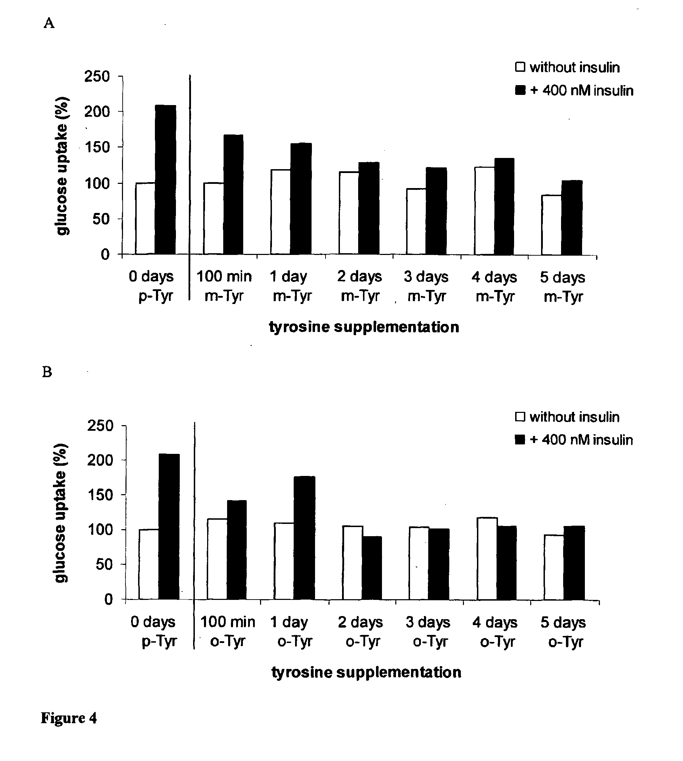

**Figure 4****.** Insulin-dependent (400 nM) uptake of deoxy-D-glucose 2-[1 2-3H(N)] into 3T3-L1 adipocytes (filled bars) in media, supplemented with para tyrosine (72 mg/l) or either meta (72 mg/l) (panel A) or ortho (72 mg/l) (panel B) tyrosine for different time periods. Open bars indicate the uptake of the insulin-untreated (control) cells, which was set to 100%. Results are representative for n=2 individual measurements.

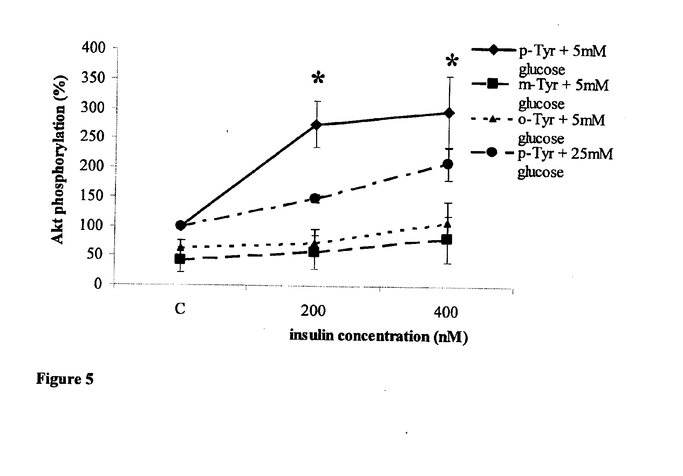

**Figure 5****.** Insulin-induced phosphorylation of Akt [protein kinase B] in 3T3-L1 adipocytes grown in media containing para-, ortho-, or meta-tyrosine in either 5 or 25 mM glucose-containing medium, at 200 and 400 nmol/l insulin treatment. Results are mean ± SEM for n=5 individual measurements. *, p<0.05 p-Tyr and 5 mM glucose vs. all other treatment groups (independent samples t-test).

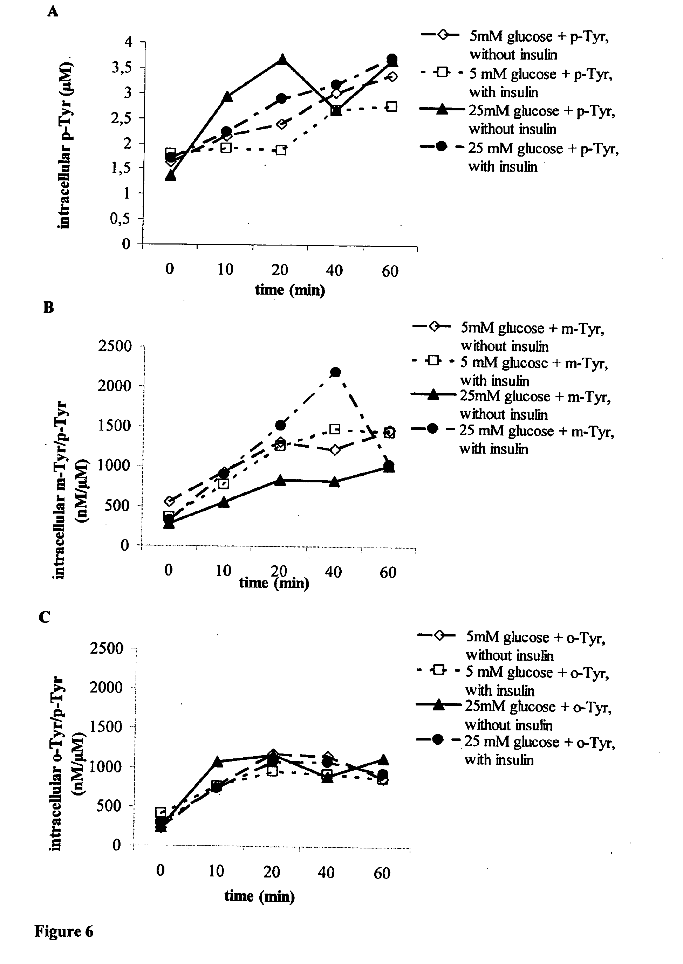

**Figure 6****.** HPLC measurement of acute uptake of para-, ortho- and meta-tyrosine by 3T3-L1 adipocytes after time-dependent incubation with the above mentioned tyrosines, with or without treatment of the cells with insulin (400 nM). Note that p-Tyr is measured in µM (panel A), while o-Tyr (panel B) and m-Tyr (panel C) are measured in nM.

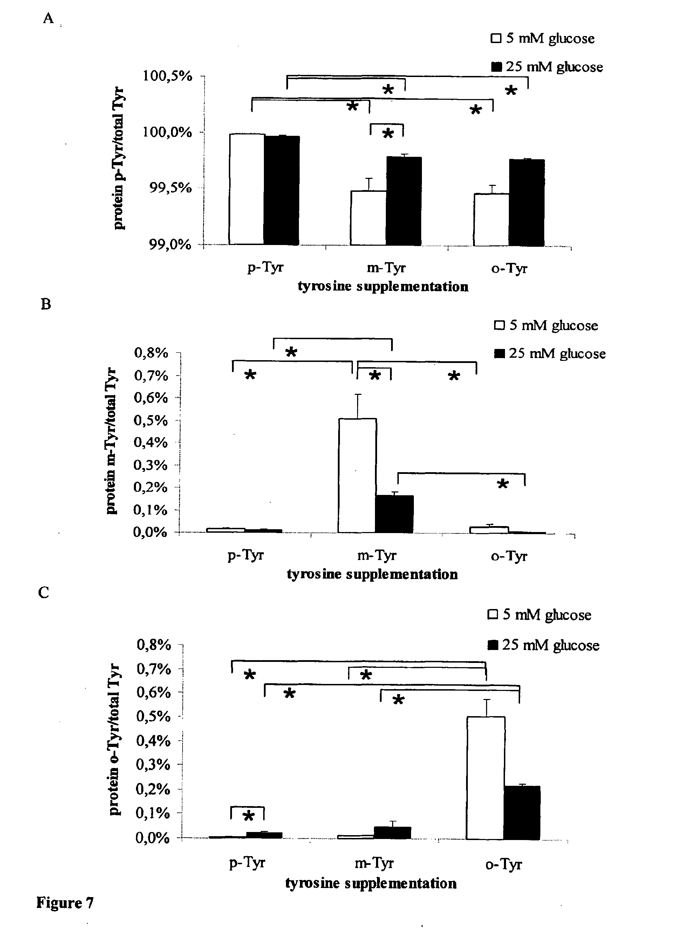

**Figure 7****.** HPLC measurement of protein-bound tyrosine isomers in cell lysates, grown in medium containing para-tyrosine (panel A), ortho-tyrosine (panel B) or meta-tyrosine (panel C). *: p<0.05 (independent samples t-test). Results are mean ± SEM for n=10 individual measurements. The amount of the amino acids was corrected to protein total tyrosine content.

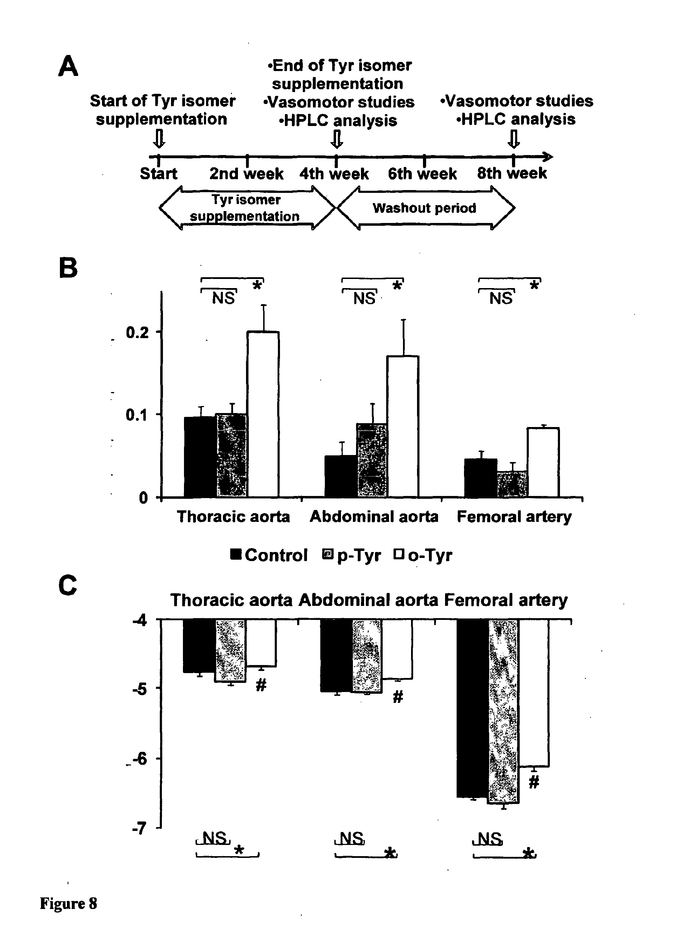

**Figure 8.A)** Experimental study protocol of tyrosine isomer supplementation in Sprague-Dawley rats. At the start, fasted, male rats (5-6 week-old) were orally supplied with either para-tyrosine (p-Tyr) or ortho-tyrosine, (o-Tyr) dissolved in saline or vehicle (saline only) for four weeks (see also in Methods section). At the end of four-week supplementation, rats were either sacrificed for HPLC and vasomotor studies or subjected to a four-week long 'washout period' by stopping the tyrosine isomer supplementation and sacrificed at the end of 8th week for HPLC and vasomotor studies. **B)** The effect of tyrosine isomer supplementation of rats on the vascular o-Tyr levels of isolated consecutive arterial segments (4th week). Sustained oral o-Tyr supplementation of rats (o-Tyr) significantly increased the o-Tyr content in all three arterial segments (i.e. thoracic and abdominal aorta, and femoral artery), while p-Tyr supplementation (p-Tyr) did not alter o-Tyr levels compared to the vehicle-treated rats (Control). [Control: Thoracic: n=4; Abdominal: n=4; Femoral: n=4; p-Tyr: Thoracic: n=4; Abdominal: n=4; Femoral: n=4; o-Tyr: Thoracic: n=4; Abdominal: n=4; Femoral: n=4]. Vascular o-Tyr levels are relative to the phenylalanine (Phe) levels. **C)** The effect of tyrosine isomer supplementation of rats on insulin-induced relaxation of isolated consecutive arterial segments (4th week). Supplementation of rats with o-Tyr (o-Tyr) markedly attenuated insulin-induced relaxations of all three isolated segments compared to the p-Tyr-treated group (p-Tyr), as well as in the abdominal aorta and femoral artery compared to the vehicle-treated rats (Control), as indicated by reduced insulin logEC50 values closer to zero. [Control: Thoracic: n=4; Abdominal: n=4; Femoral: n=4; p-Tyr: Thoracic: n=4; Abdominal: n=4; Femoral: n=4; o-Tyr: Thoracic: n=4; Abdominal: n=4; Femoral: n=4]. Data are means ± SEM. *P < 0.05; NS P > 0.05; Panel B: (ANOVA); Panel C: # P < 0.05 vs. para (Extra sum-of-squares F test)

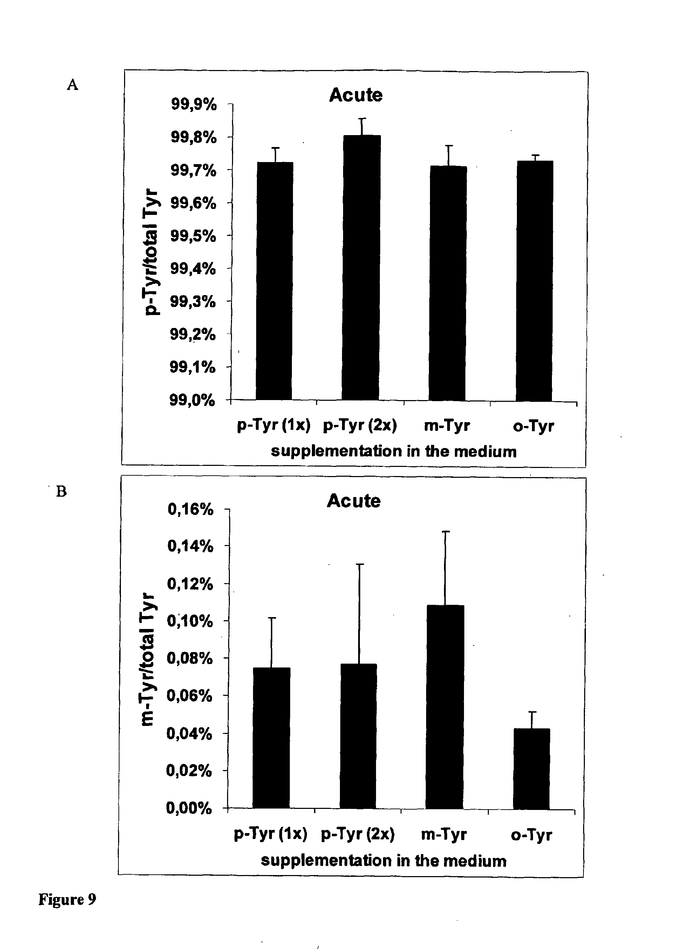

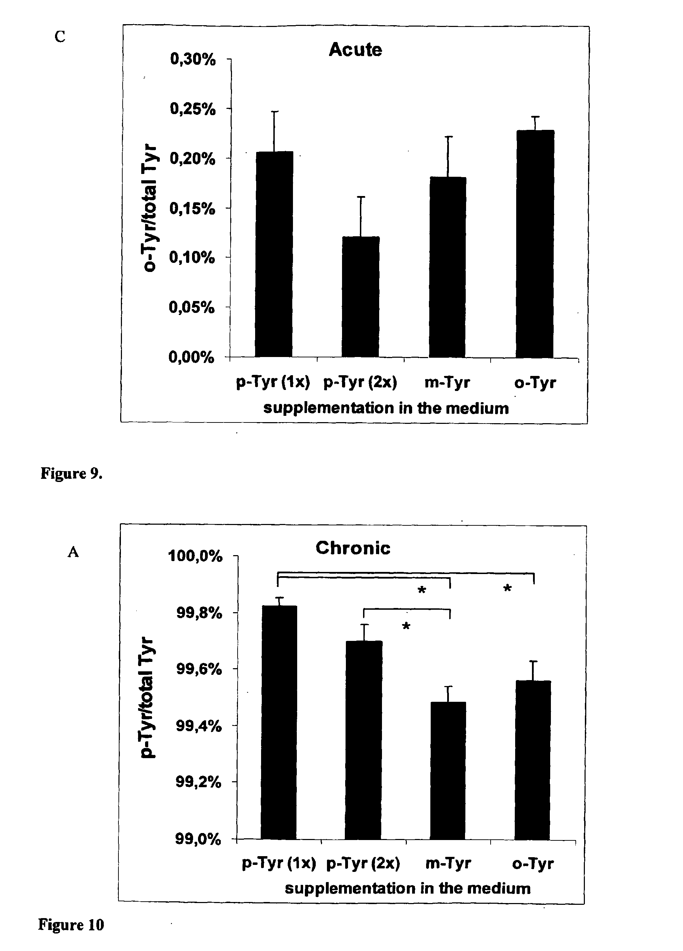

**Figure 9****.** The lack of incorporation of different tyrosine isoforms into cellular proteins of mouse endothelioma cells *at acute (30 min)* incorporation with the respective amino acids, p>0.05 for all comparisons (ANOVA). Data are in mean±SEM

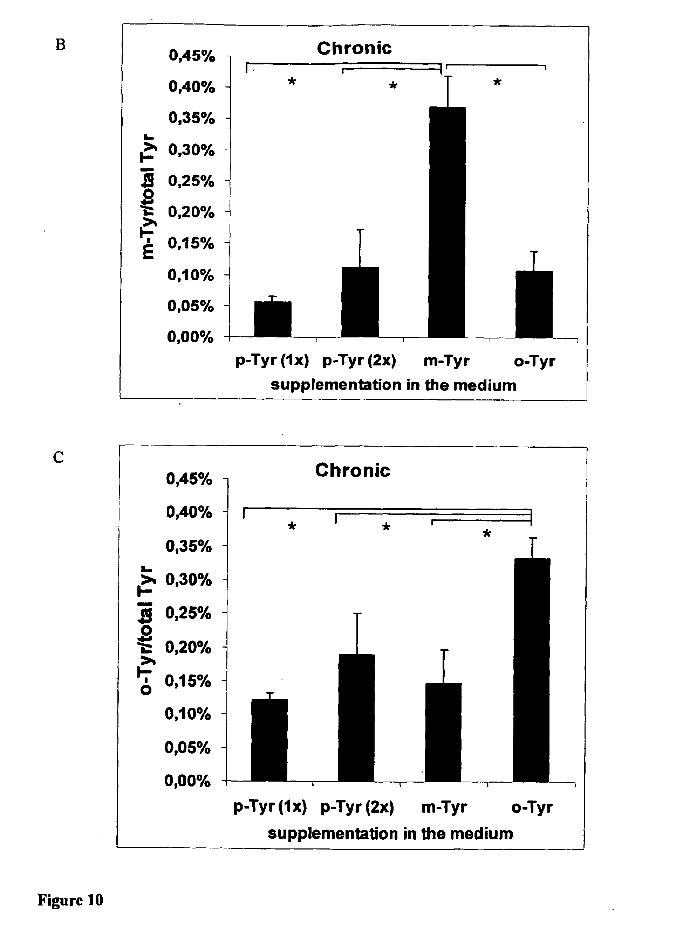

**Figure 10****.** The incorporation of different tyrosine isoforms into cellular proteins of mouse endothelioma cells *after 7 days* with the respective amino acids. *: p<0.05 (ANOVA). Data are in mean±SEM

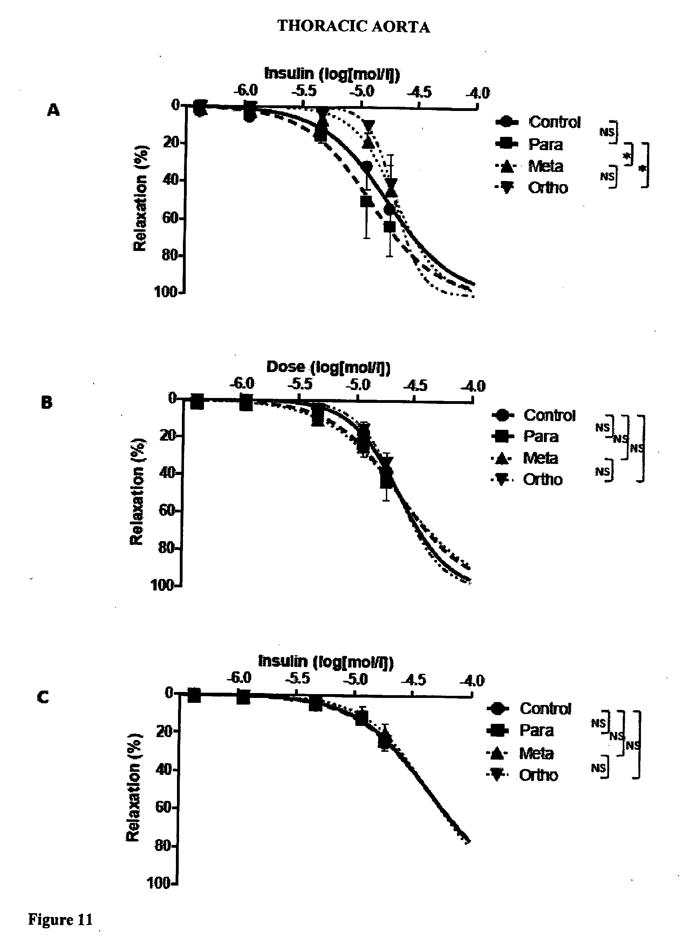

**Figure 11****.** Insulin-dependent vasorelaxation in the *thoracic aorta* of Sprague-Dawley rats fed with p-Tyr/m-Tyr/o-Tyr at 4 weeks (panel A), at the 8^{th} week (after 4 weeks wash-out, panel B) and in the acute experiment (panel C). *: p<0.05

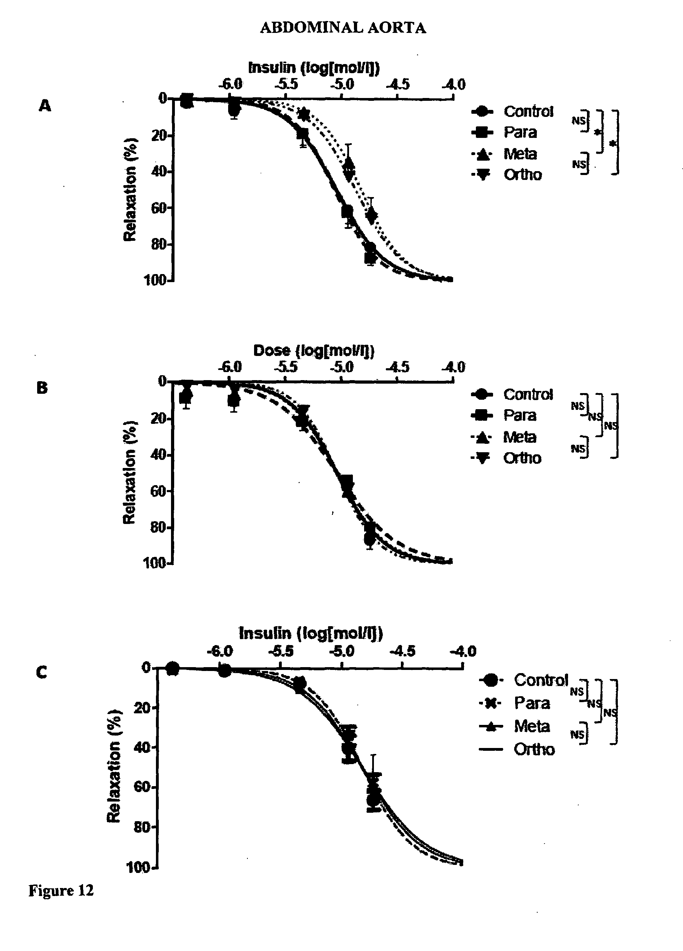

**Figure 12****.** Insulin-dependent vasorelaxation in the *abdominal aorta* of Sprague-Dawley rats fed with p-Tyr/m-Tyr/o-Tyr at 4 weeks (panel A), at the 8^{th} week (after 4 weeks wash-out, panel B) and in the acute experiment (panel C). *: p<0.05

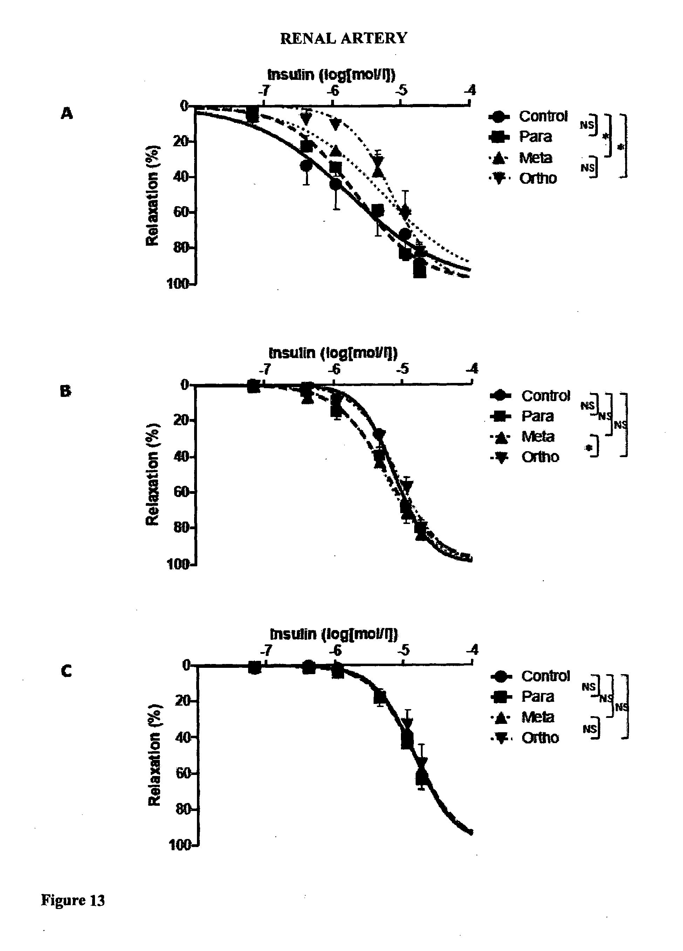

**Figure 13****.** Insulin-dependent vasorelaxation in the *renal artery* of Sprague-Dawley rats fed with p-Tyr/m-Tyr/o-Tyr at 4 weeks (panel A), at the 8^{th} week (after 4 weeks wash-out, panel B) and in the acute experiment (panel C). *: p<0.05

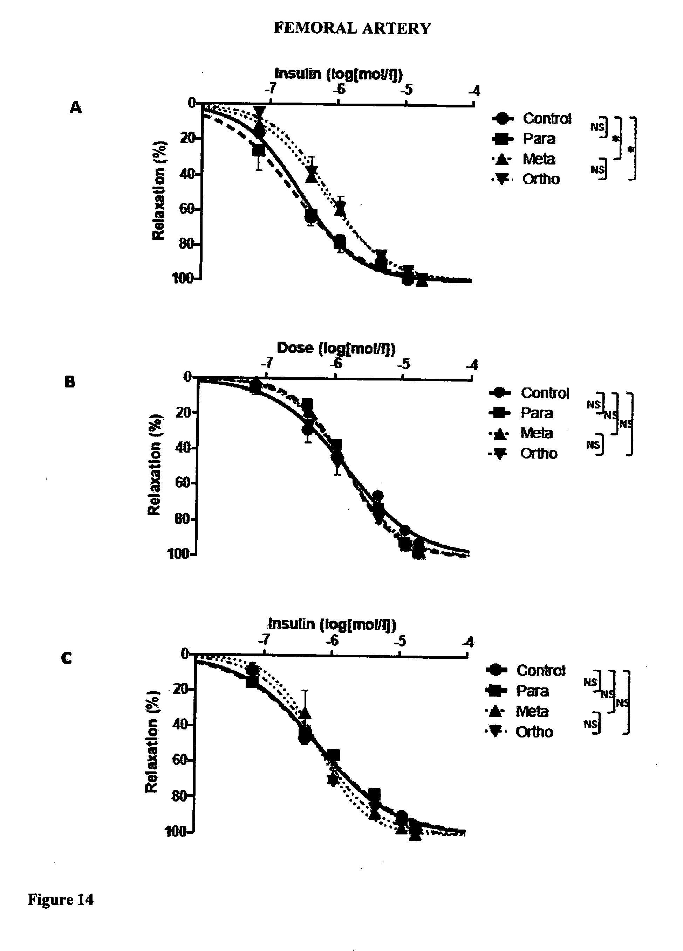

**Figure 14****.** Insulin-dependent vasorelaxation in the *femoral artery* of Sprague-Dawley rats fed with p-Tyr/m-Tyr/o-Tyr at 4 weeks (panel A), at the 8^{th} week (after 4 weeks wash-out, panel B) and in the acute experiment (panel C). *: p<0.05

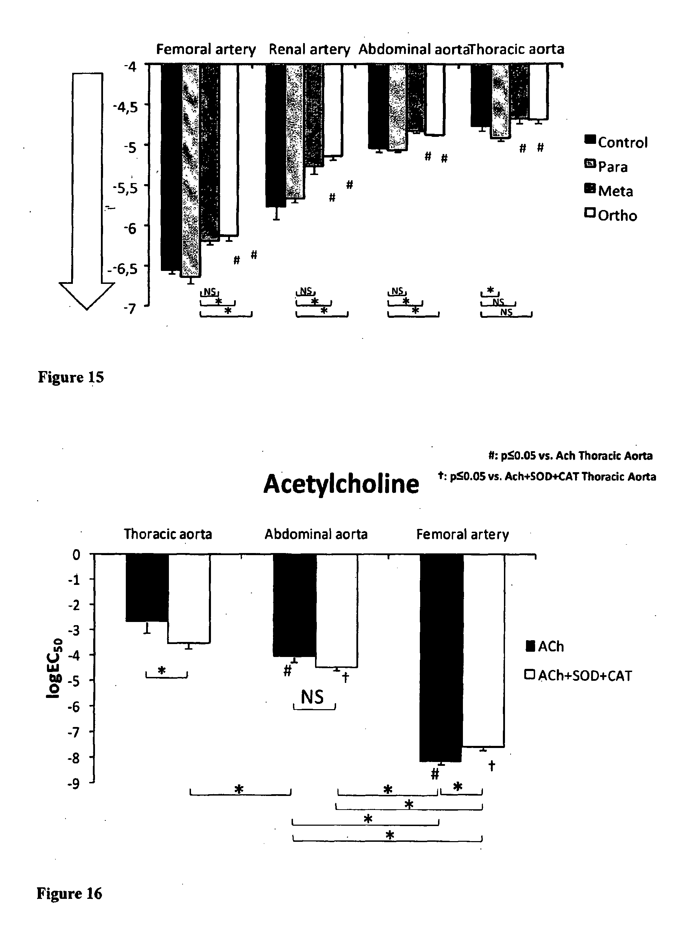

**Figure 15****.** Insulin-dependent vasorelaxation expressed as -log EC50 of the insulin dose in *different vascular segments* of Sprague-Dawley rats fed with p-Tyr/m-Tyr/o-Tyr at 4 weeks feeding. Supplementation of rats with p-Tyr (p-Tyr) increased insulin-induced relaxations only in the thoracic aorta but not in any other isolated arterial segments (i.e. abdominal aorta and femoral artery) compared to the vehicle-treated rats (Control). *: p<0.05
**Figure 16****.** Acetylcholine (Ach)-induced vasorelaxation expressed as log EC50 of the insulin dose in *different vascular segments* of Sprague-Dawley rats. In the experiment either Ach alone (black bars) or Ach plus superoxide dismutase (SOD) and catalase (CAT) was administered. *: p<0.05

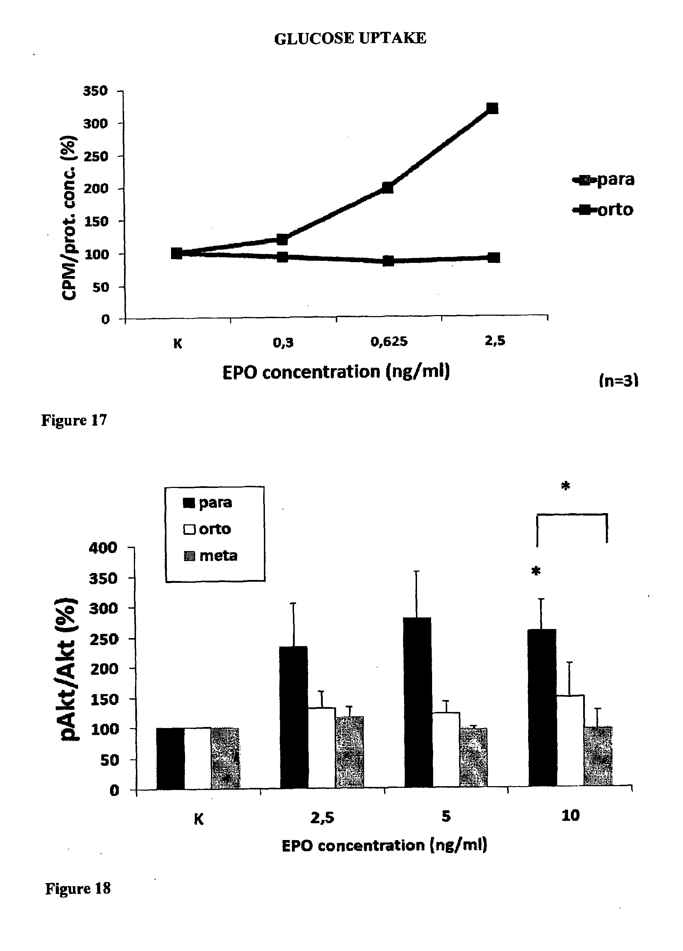

**Figure 17****.** Erythropoietin-dependent uptake of deoxy-D-glucose 2-[1 2-³H(N)] into 3T3-L1 adipocytes. Glucose uptake was measured at media containing i) para-tyrosine with 5 mM glucose content or ii) ortho-tyrosine with 5 mM. Cells were treated with various concentrations of erythropoietin (as indicated). The glucose uptake of untreated cells grown on para-tyrosine and 5 mM glucose medium was set to 100%. Results are means for n=3 individual measurements.
**Figure 18****.** Erythropoietin-dependent phosphorylation of Akt (PKB) in 3T3-L1 adipocytes. Experiments were carried out in cells grown on media containing i) para-tyrosine with 25 mM glucose content, ii) meta-tyrosin with 25 mM glucose, iii) ortho-tyrosine with 25 mM glucose. Cells were treated with various concentrations of erythropoietin (as indicated). The phosphorylation rate of untreated cells grown on para-tyrosine and 25 mM glucose medium was set to 100%. Results are mean ± SEM for n=3 individual measurements. *,p<0.05 (independent samples t-test).

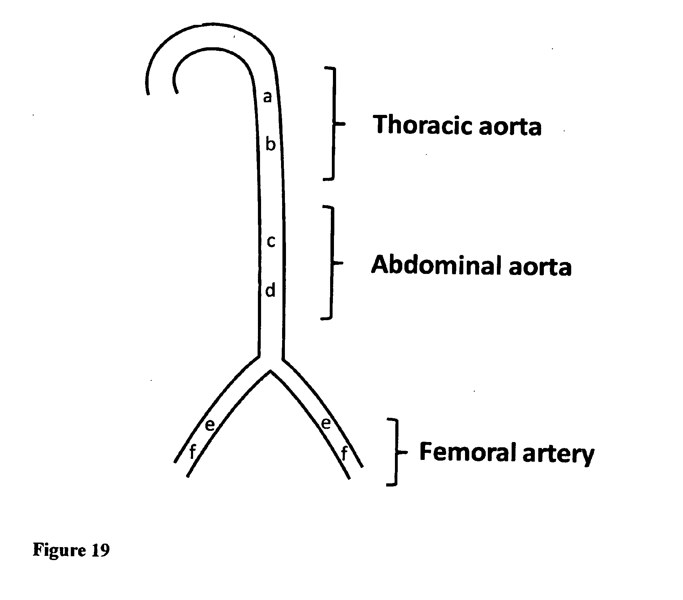

**Figure 19****.** Schematic view of consecutive arterial segments of Sprague-Dawley rats used in the experiments. The proximal segments of the thoracic (a) and abdominal aorta (c) and the femoral artery (e) were used to determine o-Tyr content with HPLC analysis. The distal parts of the same thoracic aorta (b), abdominal aorta (d) and femoral artery (f) segments were used to assess vasomotor function.

### Detailed description of the invention

Reactive oxygen species (ROS)-derived damage can be studied by the detection of stable derivatives of fatty acids, nucleic acids or amino acids. Due to the attack of the hydroxyl radical, two specific isoforms of tyrosine, namely m-Tyr and o-Tyr can be formed. These amino acids do not arise from enzymatic hydroxylation of Phe; in the latter reaction only p-Tyr is formed. Therefore, m-Tyr and o-Tyr can be regarded as specific markers of hydroxyl radical damage on Phe.

These oxidized amino acids may be produced *in loco* post-translationally by oxidation of Phe residues of protein chains, however, evidence also exists that free m- and o-Tyr can be taken up and incorporated into proteins of chinese hamster ovary cells (Gruer-Orhan2006), mouse macrophage cells (Rodgers2002) and may alter protein turnover and cellular functions.

The present inventors have performed model experiments to obtain m-Tyr- and o-Tyr-enriched proteins so as to mimic the effect of a high oxidative stress state where either Phe side-chains could be *in loco* oxidized to yield protein m- and o-Tyr side chains, or ROS-derived free m- and o-Tyr could be incorporated into proteins. When cells were grown in m- or o-Tyr containing media for 10 days, the two non-physiological amino acid isomers were incorporated into the proteins. There was no significant change in protein Phe content (data not shown).

3T3-L1 cells can be differentiated to fatty cells, and used as a model for *in vitro* effects on fatty tissue (Thomson1997). Since adipose tissue is a major locus for insulin resistance, the present inventors studied the effect of insulin on 3T3-L1 cells. The present inventors used different concentrations of the amino acid isomers in the experiments, i.e. the ratio of p-Tyr:m-Tyr or that of p-Tyr:o-Tyr varied from 1:32 to 1:1. The present inventors have found that in equimolar concentrations (72:72 mg/l), both m-Tyr and o-Tyr fully inhibit insulin-induced glucose uptake. p-Tyr needs to be present in approx. 8-32-fold higher concentration than m-Tyr or o-Tyr in the media to be able to compete with the effect of m- or o-Tyr. These data suggest a competition between the amino acids.

Incorporation of m- or o-Tyr into the proteins induces a persistent effect of oxidative stress in the patient. Thus, this means that if the cells of the patient are exposed to chronic oxidative stress the cells "remember" to the oxidative state. This occurs mainly in chronic oxidative stress leading to hormone resistance, as disclosed herein.

Thus, a typical patient to be treated according to the invention is a patient exposed to oxidative stress for a period of oxidative stress sufficient to this "oxidative stress memory" to be formed and as a consequence hormone resistance to be formed. Thus, these patients exposed to oxidative stress are at an increased risk of hormone resistance, even if the symptoms of a hormone resistance disease are not present. In these patients the treatment according to the invention can be used as a prevention of a condition or disorder due to hormone resistance induced by oxidative stress.

In case of early symptoms of a hormone resistance the condition or disorder due to hormone resistance induced by oxidative stress can be ameliorated. However, it is to be understood that even if hormone resistance according to the invention has been developed in a patient, administration of the compound for use according to the invention may still provide a prevention of the exacerbation of the disease, i.e. may prevent or alleviate downstream consequences of the said hormone resistance disease.

Thus, according to the invention a chronic oxidative stress disease or a condition, symptom, syndrome or disease induced thereby is prevented, ameliorated or treated, preferably prevented by the administration of the compound according to the invention. Preferably, the chronic oxidative stress (disease) is induced by an elevated level of OH radicals.

The state of oxidative stress can be shown or diagnosed via oxidative stress markers. Such methods are known in the art. For example, m-Tyr and o-Tyr can be regarded as specific markers of hydroxyl radical damage on Phe. It has been found that renal excretion of o-Tyr increased in patients with type 2 diabetes mellitus with or without concomitant chronic kidney disease (Molnár2005a). Furthermore, m-Tyr and o-Tyr accumulate in insoluble proteins of cataract lenses of diabetic patients (Molnár2005b). Therefore, m-Tyr and o-Tyr can be used as a marker of hydroxyl radical-induced oxidative stress and possibly a marker of insulin resistance.

A further preferred indirect marker of oxidative stress is the high-normal or elevated level of high sensitivity C-reactive protein (hsCRP). Further markers of oxidative stress are mentioned hereinbelow.

The invention is explained in more detail below via these examples and by describing the underlying scientific background and results.

Oxidative damage of different cellular constituents plays a central role in the pathogenesis of various diseases.

Thus, according to the disclosure para-L-Tyrosine (p-Tyr), p-Tyr precursor i.e. p-OH-phenyl-pyruvate or pharmaceutically acceptable salts thereof is used in a subject in the prevention, amelioration or treatment of a condition due to hormone resistance or hormone resistance disease induced by or characterized by oxidative stress.

Preferably, para-L-Tyrosine (p-Tyr), p-Tyr precursor or pharmaceutically acceptable salts thereof is used in a subject in the prevention, amelioration or treatment of erythropoietin resistance or an erythropoietin resistance disease.

Preferably, according to the disclosure para-L-Tyrosine (p-Tyr), p-Tyr precursor or pharmaceutically acceptable salts thereof is used in a subject in the prevention, amelioration or treatment of insuline resistace or an insuline resistance disease.

Preferably, according to the disclosure para-L-Tyrosine (p-Tyr), p-Tyr precursor or pharmaceutically acceptable salts thereof is used in a subject in the prevention, amelioration or treatment of acetyl-choline resistance or an acetyl-choline resistance disease.

In a large number of cases oxidative stress is one of the underlying causes of insulin resistance. There is a complex inter-relation between diabetes mellitus-related metabolic alterations, inflammation and oxidative stress. Hyperglycemia itself may lead to an increased oxidative stress via multiple pathways. One of the tissues, in which this complex interaction between ROS, AGE formation, inflammation, RAGEs etc. takes place, is adipose tissue. An *in vitro* model of adipocytes used to study insulin pathways and obesity is the 3T3-L1 cell line, a mouse embryonic fibroblast to adipose transformed cell line (Thomson 1997).

Insulin is a hormone that - under normal conditions - increases glucose uptake in fat, thus serving as the primary regulator of blood glucose concentration.

Insulin resistance is a condition in which insulin is unable to ensure that glucose is efficiently utilized by peripheral tissues, especially in the fat cells. Pathogenesis is intensively studied but not fully understood. Several pathways are suggested to play a causative role (DeFronzo2010). One potential factor implemented in the pathogenesis of insulin resistance is obesity and accumulation visceral fatty tissue.

A syndrome that is accompanied by insulin resistance is Type 2 diabetes mellitus. Furthermore, importance of insulin resistance is underlined by the fact that it may be regarded as a possible underlying cause to metabolic syndrome (Reaven 1988, Balkau 1999, Alberti 1998).

It has been proposed that periods of euglycemia during a so-called break-through could have long-term (approx. 6-12 months) effects, and some of these patients could maintain good glycemic control without the need of oral antidiabetic agents or it could delay the need for insulin. Some data also suggest that this approach was more effective in patients who were less insulin resistant (Ryan 2004, Yoshioka 2004). The background of this phenomenon has not been clarified yet, some data suggest long-term improvement of β-cell function, some rather the improved insulin resistance, some suggest the presence of both after the transient euglycemia.

It is known that insulin binding to insulin receptor triggers auto-phosphorylation of the receptor which initiates a complex signalling cascade with many key points of tyrosine phosphorylation (Chang2010). Via the phosphatidylinositol 3-kinase (PI 3-K)/Akt (protein kinase B) pathway, insulin induces metabolic effects (Glucose Transporter 4 -GLUT 4- trafficking to the cell membrane) and vasodilation (NO production), and possesses an intrinsic anti-inflammatory effect (Avogaro 2008).

Insulin can exert, however, its actions via a further pathway: involving the MAP kinase cascade that is able to promote cell proliferation. Selective impairment of the first pathway results in decreased metabolic and vasodilatory effect of insulin. Meanwhile, insulin effects are shifted towards the second pathway (i.e. towards cell proliferation), partially explaining the higher rate of malignancies observed diabetes and insulin resistance (Avogaro 2008). Therefore, an intervention wherein insulin effects via the PI3 kinase- Akt(PKB) pathway are ameliorated would result in a reverse effect, i.e. that insulin effects would be shifted from cell proliferation towards metabolic and vasodilatory activity. Therefore, said intervention could not only improve metabolic conditions and vasorectivity, but should lead to a lower rate of cell proliferation (a condition related to hormone resistance in the context of the present invention).

Insulin resistance has been associated with a reduction in the phosphorylation (activity) of insulin signalling molecules (Kanety 1995). The signalling system between the insulin receptor and Akt might be affected. In the presence of insulin resistance, insulin signalling along the PI3K/Akt pathway is selectively impaired.

Akt can be activated through different signalling ways, the effect of insulin on glucose uptake involves IRS-1, PI3K and leads to Ser(473) phosphorylation of Akt. Oxidizing agents may have an impact on insulin signalling in several ways.

Oxidative stress can influence insulin sensitivity, however, it is very important to distinguish between acute and chronic effects of oxidative stress. E.g. Wu et al showed that acute H₂O₂ burst leads to improved insulin sensitivity and signalling, whilst chronically elevated H₂O₂ levels lead to the impairment of the same signalling pathway (Wu 2005).

Long exhibition of cells to ROS causes a decreased compartment-specific activation of PI3K, resulting in decreased GLUT-4 translocation (Rudich1998). Furthermore, FFA-induced decrease in Akt phosphorylation could be reverted by siRNA against NOX2, suggesting a role of ROS in inhibition of the Akt pathway (Yuan2010). Another pathway activated by chronic oxidative stress is via JNK phosphorylation that may lead to an inhibitory Ser-phosphorylation and subsequent degradation of IRS-1 that induces inhibition of Akt-mediated signalling (Berdichevsky2010).

The enzyme catalysing Phe → p-Tyr conversion, Phe-hydroxylase is highly abundant in the kidney. Scientists of this group have shown previously that in chronic kidney disease CKD patients plasma and urinary para-tyrosine level is decreased (Molnar et al. 2005a). Furthermore, patients with CKD or renal failure preferably have hormone resistance. CKD may be a condition with lower levels of p-Tyr, and higher levels of the abnormal amino acids, m- and/or o-Tyr. Therefore the ratio of the abnormal vs. physiological amino acids i.e. m-Tyr/p-Tyr or o-Tyr/p-Tyr ratio is highly abnormal. It is feasible that patients with CKD and such abnormal amino acid proportions might especially benefit from the said intervention.

Recently, Zecchin et al. have shown that acetylcholine (ACh) induces rapid tyrosine phosphorylation and activation of Janus kinase 2 (JAK2) in rat aorta and in turn, upon, JAK2 activation, tyrosine phosphorylation of insulin receptor substrate (IRS)-1 is detected. In addition, ACh induces JAK2/IRS-1 and IRS-1/phosphatidylinositol (PI) 3-kinase associations, downstream activation of Akt/protein kinase B, endothelial cell-nitric oxide synthase (eNOS), and extracellular signal-regulated kinase (ERK)-1/2. The results support that ACh also stimulates the signal transduction pathway for IRS-1/PI 3-kinase/Akt/eNOS activation and ERK1/2 by means of JAK2 tyrosine phosphorylation in vessels. The authors suggested that in aorta of obese rats, there is not only insulin resistance but also ACh resistance, probably mediated by a common signaling pathway.

Our model aimed at mimicking a chronic high oxidative stress state, where proteins would exhibit high content of the oxidized amino acids, m- and o-Tyr. The results obtained verified that in line with findings of decreased glucose uptake, addition of m-Tyr and o-Tyr inhibited the insulin-induced Akt-phosphorylation, similarly to the high glucose environment. Therefore, inhibition of the Akt pathway by m- and o-Tyr provides an explanation of the lower glucose uptake i.e. the insulin resistant state of the fatty cells. Thus, m- and o-Tyr incorporation interferes with insulin signalling due to a disturbance of protein Tyr phoshorylation. It follows that p-Tyr, and its non-physiological analogues, m- and o-Tyr are in competition and thus the negative effect of the formation of the latter amino acids can be reversed by administration of p-Tyr and the intervention using p-Tyr could ameliorate insulin effects via the PI3 kinase- Akt(PKB) pathway. In this case insulin effects would be shifted from cell proliferation towards metabolic and vasodilatory activity. Therefore, said intervention could not only improve metabolic conditions and vasoreactivity, but may well lead to a lower rate of cell proliferation and a lower incidence of malignancies as a consequence.

Moreover, it has been shown by the present inventors that ROS has a role in ACh resistance as well. Thus, it is contemplated that p-Tyr has a beneficial effect in which ACh hyporesponsiveness effects the IRS-1/PI 3-kinase/Akt pathway.

Another example for a hormone the effect of which is impaired by oxidative stress and ROS is erythropoietin (EPO). The signal transduction of the erythropoietin receptor (EPO-R) is well described. The receptor is activated on eight cytoplasmic tyrosine domains, continued in the downstream signal of the JAK2/STAT1-5 pathway. JAK2 activity is also regulated by tyrosine-phosphorylation. Without being bound by this theory, it appears that m- or o-tyrosine misintegration into the enzymes of the signalling pathway changes the intracellular outcome of receptor activation, playing major role in EPO responsiveness.

The present inventors have applied a model of hormone resistance and found an increase of Akt and consequent increase of glucose uptake on EPO effect in 3T3-L1 adipocytes. The influence of chronic m- or o-tyrosine treatment on the effect of EPO on the glucose uptake and signal transduction of fat cells has been confirmed. According to the higher o-tyrosine level of the urine in patients of chronic kidney disease (CKD) we assumed that urine o-tyrosine level shows a negative correlation with EPO-responsiveness. A set of in vivo experiments using animal models is carried out for this purpose.

As detailed below, it was found that the underlying mechanism behind EPO resistance is similar to that of insulin resistance related to oxidative stress, and treatment by p-Tyr is contemplated. Further to p-Tyr precursors resulting in p-Tyr in the subject or organism to be treated are equally applicable.

As to exemplary conditions and diseases, anemia is a common complication in patients suffering in chronic kidney disease (CKD). Recombinant human erythropoietin (r-hu-EPO) is used in CRD since 1989, resulting in the improvement of quality of life. Approximately 10 % of the r-hu-EPO receiving subjects are hyporesponsive, associated with an increased risk of death [van der Putten et al 2008]. The reasons of EPO resistance are not clearly investigated. Van der Putten et al. highlighted the role of cytokine-inducible SH2-containing protein (CIS), upregulated by inflammatory cytokines, binds to the EPO-receptor (EPO-R) and inhibits EPO-dependent cell proliferation; or the influence of hematopoetic cell phosphatase (HCP) in the inhibition of signal transduction of EPO-R.

The present invention now provides a treatment and prevention for EPO resistant patients.

When insulin-evoked vasorelaxation in the thoracic and abdominal segment of the aorta and in the renal and femoral arteries was studied, we noticed significantly diminished response to insulin in the abdominal aorta, femoral and renal arteries of m- and o-Tyr treated animals compared to control or p-Tyr groups. The experiments suggest that while m- and o-Tyr results in insulin resistance this can be reversed by administration of p-Tyr.

As suggested above, not only p-Tyr is applicable as a competitive agent in accordance with the present invention. P-Tyr is formed also enzymatically under physiological circumstances via two pathways. On the one hand, it can by synthesized from p-OH-phenyl-pyruvate by tyrosine transaminase simultaneous conversion of L-glutamate to 2-keto-glutarate. In turn, p-OH-phenyl-pyruvate is formed from prephenate by prephenate dehyrogenase. On the other hand phenylalanine is converted to p-Tyr by phenyalanine-4-hydroxylase, therefore upstream precursors of this pathway, namely phenyl-pyruvate and prephenate are also precursors of p-Tyr.

Indicating that oxidative stress is involved in the abnormal amino acid production, hydroxyl radical converts L-phenylalanine into *m*-tyrosine and *o*-tyrosine. Thus, prephenate, phenyl-pyruvate and L-Phe are not appropriate p-Tyr precursors useful in the present invention. p-OH-phenyl-pyruvate, however, as a direct precursor of p-Tyr can be a useful substitute thereof. Preferably it is administered and supplied together with L-glutamate being a common substrate of tyrosine transaminase.

Thus, in accordance with the present invention a condition due to hormone resistance induced by oxidative stress can be prevented, ameliorated or treated by administering a compound or a pharmaceutically effective dose of a compound selected from p-Tyr and p-OH-phenyl-pyruvate or pharmaceutically acceptable salts thereof, wherein said p-OH-phenyl-pyruvate is metabolized to p-Tyr in said subject. Preferably, the condition is a condition as defined in the Brief Description of the Invention. Preferably, said condition is prevented or ameliorated, preferably prevented by administering p-Tyr or p-OH-phenyl-pyruvate.

Oxidative stress state in a patient can be assessed or diagnosed by measuring or detecting the level of an oxidative stress marker in a biological sample obtained from said patient.

Oxidative stress diseases [like diabetes mellitus (DM), chronic kidney disease (DM), obesity (OB), hypertension (DM), obstructive sleep apnea syndrome (OSAS)] lead to the formation of several reactive oxygen species (ROS) and radical-derived macromolecules (like o-Tyr). In particular, in the context of the invention, hydroxyl free radical (-OH) is formed. These species are able to activate numerous intracellular processes, among other they can lead to activation of nuclear factor kappa B (NFκB). Activation of NFκB, in turn, leads to increased expression of proinflammatory cytokines. That process results in subclinical inflammation, that can be indicated by high-normal or increased level of high-sensitivity C-reactive protein (hsCRP). As a non-binding explanation, the pro-inflammatory cytokines are able to promote activation of NADPH-oxidase via their cellular cytokine receptors. Data also suggest that CRP itself may activate NADPH-oxidase. Activation of NADPH-oxidase is responsible for increased production of ROS, i.e. oxidative stress. Hence, a vicious circle (a feed-forward or positive feed-back) may start *(**Figure 1**).* It follows that administration of the compound of the invention, while may not be sufficient to reverse the disease after hormone resistance is developed, may well prevent, either fully or partially further exacerbation thereof or may prevent, either fully or partially, the occurrence of symptoms of later phases of the hormone resistances disease.

A marker of oxidative stress if for example the level of o-Tyr, preferably as measured in the urine of patients as described by Molnár et al. (Molnár et al. 2005a). Typically, in the urine of patients exposed to oxidative stress o-Tyr level is elevated by at least 1.5 times or at least 2 times or at least 2.5 times or at least 3 times or at least 4 times or at least 5 times or at least 6 times or at least 8 times or 2 to 10 times, or 5 to 10 times or 8 to 20 times of that of the normal level or range. Typically, urinary o-Tyr excretion of patients exposed to oxidative stress o-Tyr level is at least 0.5 umol/day or at least 1 umol/day or at least 1.5 umol/day or at least 2 umol/day or at least 2.5 umol/day or 0.4 to 10 umol/day or 0.5 to 10 umol/day or 1 to 8 umol/day or 2 to 10 umol/day. The level or daily excretion of oTyr can be measured by methods known in the art. An alternative possibility is to measure m-Tyr in the urine of the patients.

Another oxidative stress marker applicable in the present invention is an increase in the inflammatory marker C-reactive protein (CRP), in particular high sensitivity CRP (hsCRP) [Abramson J L et al. (2005)]. The authors have found that oxidative stress may be a determinant of CRP levels and promote pro-atherosclerotic inflammatory processes at the earliest stages of CHD development. It has also been found that CRP levels are associated with oxidative stress whereas there are significant interrelationships among inflammation, oxidative stress, and traditional cardiovascular risk factors [Dohi J et al. (2007)]. CRP or hsCRP levels can be measured by any methods known in the art, e.g. by a method as disclosed in any of the following papers: [Abramson J L et al. (2005); Yasunari K et al. (2002); Dohi J et al. (2007)]. Typically, the hsCRP level of patients to be treated with the compound of the invention or in which prevention is to be applied show a hsCRP level of at least 50% or at least 60% or at least 70% or at least 75% or at least 90% of the upper limit of the normal range of healthy patients which shows a risk of increased oxidative stress state and thereby the development of hormone resistance. Preferably, a patient exposed to oxidative stress has a hsCRP level elevated by at least 1.2 times or at least 1.5 times or at least 2 times or at least 2.5 times or at least 3 times or at least 4 times of that of the normal level or range.

A further applicable marker of oxidative stress is the free oxygen radical test (FORT), which reflects levels of organic hydroperoxides and shows a good correlation with hsCRP level [Abramson J L et al. (2005)].

Further oxidative markers and methods for their assessment are disclosed in the following review papers: [Naito Y et al. (2010); Stephens J W. et al. (2009); Ogino K and Wang D-H. (2007)].

Formulations of p-Tyr as pharmaceutical composition or as a medicament, optionally with a pharmaceutically acceptable carrier or excipient are known in the art. Preferably, the product specification, patient leaflet or information to doctors comprises information on the condition to be treated, prevented or ameliorated with the formulation comprising the compound of the invention, as defined herein. Furthermore, formulations as a dietary supplement, food supplement, nutraceutical, functional food, food or composition are also known in the art. The invention also relates to these products for use in the prevention, amelioration or treatment of a condition as stipulated herein. In this embodiment the use of the product is indicated on the label, in a leaflet or advertisement related to said product. In particular, any of these product may be accompanies by a health claim. For example, as stipulated by Regulation (EC) No 1924/2006 of the European Parliament and of the Council of 20 December 2006 on nutrition and health claims made on foods, a "health claim" means any claim that states, suggests or implies that a relationship exists between a food category, a food or one of its constituents and health; and a particular "reduction of disease risk claim" means any health claim that states, suggests or implies that the consumption of a food category, a food or one of its constituents significantly reduces a risk factor in the development of a human disease. Regulations on similar scope and subject may be valid in other countries or geographic regions and health claims or analogous indications may suggest that the compound for use according to the invention is beneficial in a condition as disclosed herein. These products for use are within the scope of the present invention.

Preferably, the prevention, amelioration or treatment of said condition comprises, consisting of or being a part of a (personalized) regimen or administration schedule tailored to said subject.

For example, the compound can be used in a patient at risk of hormone resistance, i.e. in a condition of increased oxidative stress.

For example, the compound can be used before the symptoms of diabetes occur, e.g. for prevention of IGT and DM (monotherapy).

In diabetes the compound or the formulation can be used in combination with oral and parenteral antidiabetic agents (e.g. sulphonylureas, biguanids, glitazones, DPP-4 inhibitors, incretin mimetics, alpha-glucosidase inhibitors, glinides (meglitinides), SGLT-2 inhibitors) and with insulin for insulin resistant patients.

In patients with hypertension the compound or the formulation can be used in combination with antihypertensive agents (e.g. angiotensin converting enzyme inhibitors, angiotensin receptor blockers, direct renin inhibitors, diuretics, calcium channel blockers, alpha-1 receptor blockers, Beta-receptor blockers, direct vasodilaters, central nervous system affecting antihypertensive drugs).

Patients with hypertension can be preferably insuline resistant patients and/or patients with erythropoietine resistance and/or patients with acetyl-choline resistance.

In a preferred embodiment the daily intake of said compound is provided by administering one, two or three times the usual dose of p-Tyr or a dose as given in the brief description of the invention section or a formulation comprising an equivalent dose to the daily intake up to 30000 mg provided that preparing such a single dose is appropriate for administration and can be handled reasonably.

It is to be noted here that p-Tyr may interfere with other drugs, for example, might decrease the effectiveness of L-dopa and separation of administration of doses may be advisable. Moreover, p-Tyr may increase the effect of drugs that influence dopamine metabolism.

As a summary, we found that o- and m-Tyr causes hormone resistance for hormones of the Akt pathway, e.g. in case of insulin and erythropoietin and acetylcholine, which can be reversed by the administration of p-Tyr and its derivatives, analogues and precursors metabolised to p-Tyr in the body.

### EXAMPLES

### Materials and Methods

### Basic materials

Insulin, epinephrine, para-, meta-, ortho-tyrosine and Mg2SO4, where purchased from Sigma-Aldrich (Sigma-Aldrich, Inc., St. Louis, MO 63178, USA). NaCl, KCl, KH2PO4, NaHCO3, glucose and CaCl2*2H2O where purchased from Merck (Merck KGaA, 64271 Darmstadt, Germany).

### Cell Culture

Primary cultures of mouse embryo fibroblasts (3T3-L1) were purchased from ATCC (Manassas, US). Early passages of 3T3-L1 fibroblasts were cultured in Dulbecco's modified Eagle medium ((DMEM, Invitrogen, CAT number: 41966-029; Sigma Aldrich CAT number: D6046) supplemented with 10% Fetal Bovine Serum (Gibco, CAT number: 16170-078), 100U/ml penicillin and 0.1 mg/ml streptomycin (Gibco, CAT number: 15070-063) and 398 nM p-, m-, or o-Tyr. DMEM contained 25 or 5 mM glucose (depending on the experiment), L-glutamine, and pyruvate. Cells were grown in a humidified incubator at 37°C and 5% CO2. Differentiation medium consisted of DMEM supplemented with 10% Fetal Bovine Serum (FBS, Gibco, Csertex, Budapest, Hungary, CAT number: 10106-169) (FBS), to which a cocktail was added containing 0.5 nM isobuthylmethylxanthine (Sigma Aldrich, CAT number: I 5879), 0.17 nM insulin (Sigma Aldrich, CAT number: I 9278) and 250 nM dexamethasone (Sigma Aldrich, CAT number: 861871). From Day 4 onward, cells were maintained in DMEM /10% FBS containing 1.5 µg/ml insulin with a media change every other day until experimental treatments were initiated. For all treatments, cells were incubated overnight in serum-deprived medium after 90% of the cell population reached the adipocyte phenotype.

Experimental treatment was achieved in serum-deprived medium containing 200 or 400 nmol/l insulin, for 5 minutes. Following the treatment cells were washed twice with Saline (4°C) to remove any trace of the medium, then exposed to 80 µL lysis buffer/plate, containing 1.15% Triton X, 1M Trisbase, pH 7.4, 0.5 M EDTA, pH 8,0.2 M EGTA, pH 7, 0.1 M dithiothreitol (DTT), 5 mg/ml phenylmethylsulfonyl fluoride (PMSF), 0.1 M Na₃VO₄, 5 mg/ml leupeptin, 5 mg/ml aprotinin, Phoshatase Inhibitor Coctail 1 and 2 (Sigma Aldrich, CAT number P2850 and P5726). Adypocytes were scraped off mechanically and then frozen at ∼70°C.

Primary cultures of mouse endothelial cells (ECs) from endothelioma were purchased from LGC Promochem (Taddington, UK). ECs were cultured in Dulbecco's modified Eagle medium (DMEM; Gibco, Csertex, Budapest, Hungary) supplemented with 10% Fetal Bovine Serum (Gibco, CAT number: 16170-078), 2% mixture of penicillin-streptomycin (Gibco, CAT number: 15070-063) and 400 µmol/l p-Tyr (Control), or 800 µmol/l p-Tyr (p-Tyr), or 400 µmol/l m- and 400 µmol/l p-Tyr (m-Tyr), or 400 µmol/l o- and 400 µmol/l p-Tyr (o-Tyr). The medium was changed every 2 days. Cells were grown in a humidified incubator at 37 °C and 5% CO₂. After 7 days ECs were scraped off mechanically and then used for HPLC analysis.

### Isotope uptake

Cells were incubated in glucose free DMEM (Gibco, Csertex, Hungary) for 30 minutes, then treated with 2, 20, 200 or 400 nmol/l insulin for 100 minutes. Simultaneously 1 µCi/ml deoxy-D-glucose 2-[1 2-3H(N)] (3.7x104Bq/ml) (Izotóp Intézet, Hungary) was added to the plates for 100 minutes Cells were scraped into the medium and centrifuged for 5 minutes at 1000 rpm. The sediment was dissolved in 70 µl of lysis-buffer and glucose uptake was assessed by scintillation counting measuring 30 ul of the sample with a Beckman LS 5000 TD counter in counts per minute (CPM) for five minutes each, using an average activity as the outcome. Samples were frozen overnight at - 70 °C and protein concentration was measured using a Hitachi spectrophotometer. Results were normalized for protein content (Kaddai2009).

### Analysis of tyrosine-isomer incorporation; HPLC analysis.

Sample preparation was different depending on the type on tyrosine, aimed to be determined. Methods were based on earlier publications with modifications (Molnár2005a, Molnár2005b) To measure the total protein-bound cellular tyrosine content, after adding 200 µl of distilled water, samples were frozen overnight at - 70 °C to achieve cell lysis. After melting up and centrifugation at 4000 rpm, for 10 minutes, 200 µl, 60% trichloroacetic acid was added to 200 µl supernatant and was incubated on ice for 30 minutes, to precipitate proteins. Following the second centrifugation at 4000 rpm, for 10 minutes, sediment was resuspended in 1% trichloroacetic acid and 4 µl of 400 mmol/l desferrioxamine and 40 µl of 500 mmol/l butylated hydroxytoluene were added to the samples to avoid possible free radical formation during hydrolysis. Then 200 µl or 400 µl of 6N hydrochloric acid was added, and we performed an overnight acid hydrolysis of the proteins at 120 °C. The hydrolyzate was then filtered through a 0.2 µm filter (Millipore Co., Billerica, MA, USA) and 20 µl of the filtrate was injected onto the HPLC column of a Shimadzu Class LC-10 ADVP HPLC system (Shimadzu USA Manufacturing Inc., Canby, OR, USA) using a Rheodyne manual injector.

Total protein-bound cellular tyrosine content of the ECs was measured by a similar method with the following differences: After adding 200 µl of distilled water, samples were sonicated 2 minutes long with ultrasonic homogenizer to achieve cell lysis. Addition of 100 µl, 60% trichloroacetic acid was followed by centrifugation and resuspension as described above. After resuspension 100 µl, 60% trichloroacetic acid was added. Following the second centrifugation as described above, previous process was repeated once again. Finally sediment 4 µl of 400 mmol/l desferrioxamine and 40 µl of 500 mmol/l butylated hydroxytoluene were added to the sediment to avoid possible free radical formation during hydrolysis. Then 400 µl of 6N hydrochloric acid was added to the samples.

To measure the total intracellular non-protein-bound tyrosine content, 200 µl of distilled water was added to the samples before they were frozen overnight at - 70 °C to achieve cell lysis. Samples were melt up and centrifuged for 15 minutes, at 15 000 rpm. 200 µl of 60 % trichloroacetic acid was added to 200 µl of the supernatant. Following 30 minutes incubation on ice, samples were centrifuged again, for 15 minutes, at 15,000 rpm. The supernatant was filtered in the above mentioned way, was diluted 5 times, and 160 µl distilled water was added to 40 µl of filtrate, which was injected onto the HPLC column.

For the determination of tyrosine content of the vascular wall, the modified method of Molnar et al. (Molnár2005b) was used. The proximal sections were hydrolyzed in well-closing, O-ring protected polypropylene tubes. Four µl of 400 mmol/l desferrioxamine (final concentration: 3.6 mmol/l) and 40 µl of 500 mmol/l butylated hydroxytoluene (final concentration: 45 mmol/l) were added to the samples. Then 200 µl of 6N hydrochloric acid was added to the samples.

We performed an overnight acid hydrolysis of the proteins obtained from ECs and arteries at 120 °C. The hydrolyzate was then filtered through a 02 µm filter (Millipore Co., Billerica, MA, USA) and 20 µl of the filtrate was injected onto the HPLC column.

Autofluorescence of p-, m- and o-Tyr and Phe was used to measure their amount in the samples. Thus, no staining or derivatisation was needed. Samples were run on a Shimadzu Class 10aDVP HPLC system equipped with a RF-10 AXL fluorescent detector (Shimadzu USA Manufacturing Inc., Canby, OR, USA) optionally using a Rheodyne manual injector. The mobile phase consisted of 1 % acetic acid and 1 % sodium-acetate dissolved in water, the separation took place on a LiChroCHART 250-4 column (Merck KGaA, 64271 Darmstadt, Germany), in an isocratic run. 275 nm excitation and 305 nm emission wavelengths were used to measure p-, m- and o-Tyr, while Phe was detected at 258 nm excitation and 288 nm emission wavelengths. Determination of the area under-the-curve (AUC) and external standard calibration were used to calculate exact concentrations of the amino acids. In some cases the elution time of the substances was also verified by standard peak-addition method. The amino acid concentrations were corrected for total tyrosine or phenylalanine concentrations.

### Western blot analysis

The samples of EC lysates were sonicated (2 min) and centrifuged (10 min, 13,000 rpm, at 4°C). Protein content of samples was determined by the Bradford method using bovine serum albumin as a standard Bio-Rad Benchmark Plus. Samples were solubilized in a buffer of 100 mM Tris-HCl (pH 6.8), 4.0% sodium dodecyl sulphate (SDS), 20% glycerol, 200 mM DTT, and 0.2% bromophenol blue. Samples (80 to 120 µg protein) were electrophoretically resolved on 10% polyacrylamide gels and transferred in a buffer (pH 8.3) containing 38 mM glycine, 48 mM Tris-base, and 20% methanol to PVDF membranes (Amersham-Biotech, AP Hungary, Budapest, Hungary) for 90 min at 250 mA.

Membranes were incubated overnight with the immunoglobulin G (IgG) monoclonal antibodies diluted to 1:1000 in TBS-T (0.1%), containing 5% bovine serum (BS). Antibody for anti-phospho-(Ser473)-Akt (pAkt, Ser473, #9271, 1:1000, Cell Signalling Technology, Beverly, USA) was used first. Membranes were washed with TBS-T 0.1% and incubated in peroxidase conjugated IgG secondary antibody diluted in 5% non fat milk to 1:2000 with anti-rabbit polyclonal (Cell Signaling Technology, #7074) to detect anti-phospho-(Ser473)-Akt. To reprobe Western blots with alternative primary antibodies for total total PKB/Akt (#9272, Cell Signaling Technology, Beverly USA), membranes were stripped as follows: membranes were washed in TBS-T 0.1% for 10 minutes, then were put in stripping buffer, containing 1.5% glycine, 0.1% SDS, 1% Tween-20 at ph:2.2, for 2x10 min, then washed in destillated water. Blots were detected using enhanced chemiluminescence (ECL; Pierce Biotech, Bio-Rad, Budapest, Hungary). Computerized densitometry (integrated optical density) of the specific bands was analyzed with Scion Image for Windows Software. Protein expressions were corrected for total Akt protein levels and were adjusted for controls.

Routine blood and urine tests were carried out by the Department of Laboratory Medicine at the University of Pécs Medical School according to standard clinical laboratory procedures.

### Animals and tissue preparation

The experiments were carried out with the permission of the Hungarian Local Animal Experiment Committee. Shortly after weaning male CFY rats (a strain of the Sprague-Dawley rat) were placed in individual cages and held on regular diet. After two hours of fasting animals (4-5 week-old, 80-140g) were loaded with 1.76 mg/die para-, meta- or ortho-tyrosine (p-, m-, o-tyr; Sigma-Aldrich, Inc., St. Louis, MO 63178, USA) dissolved in saline or vehicle (saline only) by gavage (per os) six days of the week. After one month treatment, animals were either anaesthetized with ether and were decapitated with a guillotine or p-, m-, o-tyr and vehicle supplementation was discontinued ("washout" period) and they were sacrificed two weeks later (Figure 8.A). The descending thoracic and abdominal aorta, renal and femoral arteries were removed and cleaned from connective tissue (Figure 19). Sections were immediately hydrolyzed for HPLC analysis or were used for vascular reactivity studies.

In further tyrosine isomer incorporation studies when effects of chronic oral p-Tyr and o-Tyr supplementation of rats on the o-Tyr level and insulin-induced relaxation of isolated arterial segments were studied, 18 Male Sprague-Dawley rats (5-6 week-old) were used. Rats were orally supplied with either 1.76 mg/die of p-Tyr or o-Tyr or vehicle during six days per week for four weeks. Rats treated with p-Tyr, o-Tyr or vehicle nearly tripled their body weight, but there was no difference between the groups. At the end of four-week treatment, HPLC and vasomotor studies were performed. In another group of rats, p-Tyr/o-Tyr supplementation was discontinued for four weeks ("washout" period), after which they were sacrificed for HPLC and vasomotor studies.

Preparation of consecutive arterial vessels is indicated in Figure 19.

### Vascular reactivity studies

The modified method of Fésüs et al. (Fesüs2007) was used. The distal parts of the vessels were dissected into 2 mm long segments in ice-cold Krebs buffer and rings were mounted on two stainless steel wires (40µm in diameter) in a Danish Multimyograph Model 610M (DMT-USA Inc., Atlanta, GA, USA). Vessels were bathed in Krebs buffer (containing 119mM NaCl, 4.7mM KCl, 1.2mM KH₂PO₄, 25mM NaHCO₃, 1.2mM Mg₂SO₄, 11.1 mM glucose, 1.6mM CaCl₂*2H₂O, pH 7.4) and gassed with 5% CO₂ and 95% O₂ at 37°C. The resting tension/internal circumference relationship for each vessel was determined and then the internal circumference was set to 0.9 x L100, where L100 is the internal circumference the vessel would have had in vivo when relaxed under a transmural pressure of 100 mmHg. After this normalization procedure vessels were allowed to stabilize for 30 min, and then isometric tension was continuously recorded. Rings were preconstricted with 100nM epinephrine. After reaching a stabile contraction plateau, relaxant responses to increasing doses of insulin were assessed. The rate of relaxation was expressed as percentage of the contraction evoked by epinephrine 100%). Para-, meta-, ortho-tyrosine or vehicle were added to the vessel chamber 10 minutes before the addition of epinephrine and 30 minutes before the addition of insulin.

In a variant of the method, p-, m- and o-Tyr are used separately in the experiments. In a further variant of the method either p-, and o-Tyr or m- and o-Tyr are used together and the results are compared to data obtained when the compounds are used separately so as to evaluate competitive effect.

### Statistics

Statistical significance was calculated using Student's t-test or ANOVA as appropriate. All distributions were normal, data are expressed as means ± SEM. Tests were performed typically with ANOVA, extra sum-of-squares F test, non-parametric tests as appropriate using the SPSS program package, Version 15.0 (SPSS Inc., Chicago, IL, USA), and GraphPad Prism 5 (GraphPad Software Inc., La Jolla, CA, USA), considering P values of 0.05 or less to be significant.

### RESULTS

### Uptake of isotope-Iabelled 2-deory-D-glucose in 3T3-L1 cells

We measured the uptake of isotope-labelled 2-deoxy-D-glucose in 3T3-L1 cells. In cells grown on medium containing p-Tyr + 5 mM glucose, increasing concentrations of insulin lead to an increase in deoxy-D-glucose uptake. In cells grown on medium containing p-Tyr + high glucose (25 mM), insulin failed to increase glucose uptake. Similar to that, no insulin effect could be verified in cells grown on medium with normal glucose (5 mM) but with m-Tyr or o-Tyr supplementation (*Figure 2*). Cells were grown for 11 days in each experiment.

The ability of m-Tyr and o-Tyr to inhibit insulin-induced glucose uptake was found to be concentration-dependent. While in cells grown on p-Tyr and normal glucose, insulin (400 nM) lead to an approx. 2-fold increase in glucose uptake (white and first black bar), with increasing concentrations of m-Tyr (*Figure 3A*) and o-Tyr (*Figure 3B*), the insulin-induced glucose uptake decreased. At equimolar concentrations of m-Tyr and p-Tyr or o-Tyr and p-Tyr (72-72 mg/l), insulin had no effect on glucose uptake at all (last bars), suggesting a strong competition of the three amino acids.

From the figure it can be seen that p-Tyr shall be present in approx. 8-32-fold higher concentration than m-Tyr or o-Tyr in the media to be able to effectively compete with m-Tyr or o-Tyr.

We also tested whether the effect of m-Tyr and o-Tyr was time-dependent. We found that baseline glucose uptake did not show a marked change in 3T3-L1 cells grown on m-Tyr- or o-Tyr-containing medium. However the insulin-induced glucose uptake was blocked by m-Tyr and o-Tyr, in a time-dependent manner (*Figure 4A and 4B*).

### Effect of m-Tyr and o-Tyr on the insulin signal transduction pathway

We investigated the effect of addition of m-Tyr and o-Tyr to the media of the cells on different signal transduction pathways. We found that in cells grown on p-Tyr and low glucose, increasing doses of insulin lead to a 3-fold increase in Akt (protein kinase B) phosphorylation compared to controls. In cells grown on high glucose media with p-Tyr only, insulin induced a significantly lower Akt-phosphorylation. In cells grown on m-Tyr or o-Tyr, Akt phosphorylation could not be significantly enhanced by insulin (*Figure 5*).

### Acute uptake of p-Tyr, m-Tyr and o-Tyr

In the background of the above findings we hypothesized that the supplied amino acids can enter the cells. This hypothesis was also tested: after different incubation times the intracellular total p-Tyr, m-Tyr and o-Tyr content was measured using HPLC. We found that supplementation with p-Tyr *(**Figure 6A**)*, m-Tyr *(**Figure 6B**)* or o-Tyr *(**Figure 6C**)* could efficiently elevate the levels of the respective amino acid in the 3T3 cells, both in normal and in high glucose media, as well as with and without insulin treatment.

### Incorporation of m-Tyr and o-Tyr into cellular proteins

It was also tested, whether longer-term administration (11 days) of m-Tyr or o-Tyr supply leads to an increase of protein-incorporated m-Tyr and o-Tyr. p-Tyr content was expressed as percentage of total protein tyrosine content, i.e. p-Tyr/[(p-Tyr)+(m-Tyr)+(o-Tyr)] ratio was calculated. m- and o-Tyr were normalized for total Tyr content, as well. Tests were carried out both in normal (5mM) and in high glucose (25 mM) experimental settings. Growing the cells on m-Tyr or o-Tyr supplemented medium lead to a decrease in protein p-Tyr levels both under normal and high glucose conditions. p-Tyr content was significantly higher in m-Tyr supplied cells grown in high glucose medium compared to normal glucose medium *(**Figure 7A**).* m-Tyr content of the cells grown on m-Tyr-supplied medium was significantly higher than in cells grown on p- or o-Tyr, both in 5 mM and 25 mM glucose media. In cells grown in m-Tyr medium, protein m-Tyr content was significantly lower in high glucose environment compared to normal glucose medium *(**Figure 7B**).* When investigating the protein o-Tyr content, we found that it was significantly higher in p-Tyr-supplied cells grown in high glucose medium than in cells grown in 5 mM glucose medium. In o-Tyr supplied cells, protein o-Tyr was significantly higher compared to p-Tyr or m-Tyr supplied cells, both in normal and high glucose media *(**Figure 7C**).*

### Arterial presence of o-Tyr and its effects on vasoactivity

In a further experiment, the descending thoracic and abdominal aorta and femoral arteries of untreated rats were removed, cleaned and used for assessment, as described in the Materials and Methods chapter. Sections were immediately hydrolyzed for HPLC analysis or were used for vascular reactivity studies. The baseline o-Tyr content of fasting animals (as a measure of oxidative stress) of different vessels showed a rank order of thoracic aorta > abdominal aorta > femoral artery *(**Figure 8**, panel B, black bars)* and the response to insulin showed the opposite rank order of thoracic aorta < abdominal aorta < femoral artery *(**Figure 8**, panel C, black bars; the difference were significant).*

### The impact of supplementation on the levels of meta- and ortho-tyrosine in the vascular wall

In this experiment rats were loaded with para-, meta- or ortho-tyrosine. After one month treatment, animals were either sacrificed or supplementation was discontinued ("washout' period) and they were sacrificed four weeks later *(**Figure 8**, panel A).* After one month treatment with p-, m-, o-Tyr and vehicle we measured the tyrosine content of the thoracic and abdominal part of the aorta and the renal and femoral arteries. In every examined artery we observed significantly higher o-Tyr level in the group treated with o-Tyr compared to the control group *(**Figure 8**, panel B, white bars).* However, in this experiment no significant difference was found between the m-Tyr content of different groups (data not shown).

We determined whether sustained *in vivo* supplementation of rats with o-Tyr had any impact on insulin-induced relaxation *ex vivo.* We obtained significantly diminished response to insulin in the abdominal aorta and femoral artery of o-Tyr-treated rats *(**Figure 8**, panel C, white bars)* compared to the vehicle- *(black bars)* or the p-Tyre groups *(grey bars).* In the thoracic aorta, there was no significant difference between the control and o-Tyr-treated groups. In contrast, we found increased relaxation in response to insulin in the thoracic aorta isolated from p-Tyr-treated rats compared to other groups *(**Figure 8C**).* Four weeks after the discontinuation of tyrosine supplementation (8^{th} week) the o- and m-Tyr content of the same vessels were assessed. There was no significant difference between the o- and m-Tyr content of different groups (data not shown).

### Incorporation of para-, meta- and ortho-tyrosine into cellular proteins of endotheliel cells

Hormones evoke vasoactive effect inducing responsiveness of the endothelial cells. Thus, the changes of the amino acid composition of these cells may have key role. In an acute experiment, 30 minutes incubation with the respective amino acids did not lead to a significant change in cellular protein p-, m- and o-Tyr content *(**Figure 9A**, B and C).*

In cellular proteins of ECs grown on m-Tyr for 7 days, there was a higher relative content of *m-Tyr* compared to p-Tyr supplemented or control cells *(**Figure 10 B)**.* Similarly, in the proteins of o-Tyr treated cells higher o-Tyr content was measured compared to p-Tyr or control ECs *(**Figure 10* *C*). Relative p-Tyr amounts barely reflected changes in m- and o-Tyr content *(**Figure 10 A)**.*

### The impact of para-, meta- and ortho-tyrosine supplementation on insulin-induced vasorelaxation

After one month treatment *(**Figures 11 to 14**, Panels A),* and thereafter one month washout *(Panels B)* and in an acute experiment, i.e. after 30 minutes incubation *(Panels C)* we assessed the insulin-evoked vasorelaxation in the thoracic and abdominal segment of the aorta and in the renal and femoral arteries *(**Figures 11 to 14**).* In the *thoracic aorta,* which showed the highest level of o-tyr reflecting the highest intravascular-intramural oxidative stress among the four vessels studied *(**Figure 8**, panel B, black bars),* we did not observe any significant further decline of response to insulin with supplementation by m-, or o-Tyr at week 4 *(**Figure 11A**).* In contrast, there was a higher vasorelaxation response to insulin in the thoracic aorta of the p-Tyr supplemented group compared to the other groups suggesting a real competition of the amino acids *(**Figure 11A**).* We noticed significantly diminished response to insulin in the lower oxidative state segment (abdominal aorta and femoral arteries, see *Figure 8* *panel B)* of animals supplemented with m- and o-Tyr compared to control animals *(Panels A of* *Figures 12**,* *13* *and* *14**).* Furthermore, in these arteries we could not detect significant difference between the control and p-Tyr and between the m- and o-Tyr groups *(**Figures 12A**,* *13A* *and* *14A**).* The chronic effects were completely abolished by 4 weeks washout *(Panel B of* *Figures 11**,* *12**,* *13* *and* *14**)* in all segments.

In the acute experiment, after 30 minutes incubation with the respective amino acids, vasoralaxation remained unchanged *(Panels C of* *Figures 11**,* *12**,* *13* *and* *14**).*

Calculated logEC50 values of these experiments at the 4^{th} week (chronic p-, m- and o-Tyr supplementation) are depicted in *Figure 15**.*

### Reversal of the effect of endogenous meta- and ortho-tyrosine by para-thyrosine supplementation in insulin-induced vasorelaxation experiments in the thoracic aorta

As seen on Figure 15, chronic p-Tyr supplementation has led to a greater vasorelaxation in the thoracic aorta as compared to the same vessel of the control animals (note the significant difference between the black and first grey column of thoracic aorta on Figure 15). This data suggests that even in the control animals, there is a chronic baseline oxidative stress resulting in lower insulin sensitivity that can be partially reverted by the administration of p-Tyr.

It appears that the level of o-Tyr is the highest in the thoracic aorta which is in correlation with hormone resistance. Therefore, it is suggested by this experiment that loading of thoracic aorta by p-Tyr results in a partial replacement of o-Tyr and an improved vasorelaxation. This works as a model of reversal of hormone resistance related effect caused by oxidative stress in the aorta.

In a similar further proposed experiment sustained in vivo supplementation or rats with o-Tyr and p-Tyr is carried out and response to insulin is examined in the same isolated arterial segments. It is expected that diminished response to insulin is reversed. Metabolic effects of m- and o-Tyr supplementation could also be studied by using functional imaging. Also, potential beneficial effects of p-Tyr supplementation could be investigated in spontaneous hypertensive rats, Zucker fatty diabetic rats, rats with angiotensin-II minipumps or rats undergoing 5/6 nephrectomy.

### Effect of short-term antioxidant treatment on acetylcholine-induced vasorelaxation

Vasorelaxation experiments with acetylcholine were performed. In case of Ach-induced vasorelaxation, we found a significant difference between the different vascular segments, i.e. vasorelaxation by Ach was easiest achieved in the femoral arteries, sensitivity to Ach was significantly lower in the abdominal and even lower in the thoracic aorta. Treatment with SOD and catalase resulted in a small but significant change in the case of the thoracic aorta and the femoral artery, however the difference in EC50 values of the different segments remained significant (Figure 16). These data suggest that the substantial difference in Ach-induced vasorelaxation remains the same after SOD+CAT treatment, thus it is not an acute oxidative stress that regulates the vasorelaxation.

This experiment is to be continued by Tyr supplementation in analogy with the setting described in the "The impact of para-, meta- and ortho-tyrosine supplementation on insulin-induced vasorelaxation" section above and a similar effect is expected.

### Uptake of isotope-labelled 2-deoxy-D-glucose in 3T3-L1 cells by erythropoietin (EPO)

In a previous experiment we found an increase of Akt phosphorylation and consequent increase of glucose uptake in 3T3-L1 adipocytes. In the present experiment we measured the uptake of isotope-labelled 2-deoxy-D-glucose in 3T3-L1 cells in the presence of para- and ortho-Tyr at various concentrations of EPO *(**Figure 17**).* We found that EPO-induced glucose uptake in the presence of ortho-tyrosine was inhibited compared to the para-Tyr control.

### Akt phosphorylation in erythropoietin signalling

An alteration in Akt phosphorylation due to a downstream effect of EPO has been studied in 3T3-L1 adipocytes by Western blot in the presence of para-, ortho and meta-tyrosine *(**Figure 18**)*. The presence of ortho- and meta-Tyr in the medium inhibited the normal increase in the Akt phosphorylation.

### Setting a p-Tyr treatment regimen

As an example, a dyslipidemic patient with a risk of metabolic syndrome having a body mass index of eg. 29 attends regular medical control. para-L-Tyrosine therapy is started with a daily intake of 600 mg in three doses 200 mg each. Serum and urinary levels of p-Tyr, o-Tyr and m-Tyr is measured in every two weeks together with usual blood parameters including serum insulin and glucose. A low-fat low glucose diet is proposed and kept by the patient. p-Tyr doses are increased in the knowledge of the results to an effective level.

As a further example, a male patient attends medical control with a complaint of high blood pressure and increased stress at regular daily work in the last month. In this situation he relates an increased intake of coffein and sugar. His high sensitivity C-reactive protein (hsCRP) level is measured as well as his serum insulin, and plasma glucose level. Each measured values were found to be close to the upper limit of the normal range while plasma glucose slightly exceeded it. p-Tyr therapy is started with a daily intake of 3 g (three times I g doses) and in a month all the parameters are measured again and found to be somewhat diminished p-Tyr therapy is continued and in a further month the values returned to the medium normal range. p-Tyr therapy continued with a daily intake of 1.5 g in three doses.

### INDUSTRIAL APPLICABILITY

The present invention provides compounds, formulations and methods for use in the treatment of hormone resistance disorders and chronic oxidative stress disorders.

### REFERENCES

Abramson J L et al. (2005) Association between novel oxidative stress markers and C-reactive protein among adults without clinical coronary heart disease. Atherosclerosis 178 (2005) 115-121
Alberti KG, Zimmet PZ. Definition, diagnosis and classification of diabetes mellitus and its complications. Part 1: Diagnosis and classification of diabetes mellitus provisional report of a WHO consultation. Diabet Med. 1998;15:539-553.
Avogaro A, de Kreutzenberg SV, Fadini GP Oxidative stress and vascular disease in diabetes: is the dichotomization of insulin signaling still valid? Free Radic Biol Med 2008; 44: 1209-15.
Balkau B, Charles MA: Comment on the provisional report from the WHO consultation. European Group for the Study of Insulin Resistance (EGIR) Diabet Med. 1999 May;16(5):442-3.
Berdichevsky A, Guarente L, Bose A: Acute oxidative stress can reverse insulin resistance by inactivation of cytoplasmic JNK. J Biol Chem. 2010 Jul 9;285(28):21581-9.
Brasnyó P, Molnár GA, Mohás M, Markó L, Laczy B, Cseh J, Mikolás E, Szijártó IA, Mérei Á, Halmai R, Mészáros LG, Sümegi B, Wittmann I: Resveratrol improves insulin sensitivity, reduces oxidative stress and activates the Akt pathway in type 2 diabetic patients. Br J Nutr. 2011 Mar 9:1-7. [Epub ahead of print]
Chang YC, Chuang LM. The role of oxidative stress in the pathogenesis of type 2 diabetes: from molecular mechanism to clinical implication. Am J Transl Res. 2010 Jun 10;2(3):316-31.
DeFronzo RA: Insulin resistance, lipotoxicity, type 2 diabetes and atherosclerosis: the missing links. The Claude Bernard Lecture 2009. Diabetologia. 2010 Jul;53(7):1270-87.
Dohi J et al. (2007) Association among C-reactive protein, oxidative stress, and traditional risk factors in healthy Japanese subjects. International Journal of Cardiology 115 63 - 66.
Fernstrom JD, Fernstrom MH: Monoamines and protein intake: are control mechanisms designed to monitor a threshold intake or a set point? Nutr Rev. 2001 Aug;59(8 Pt 2):S60-5; discussion S66-8.
Fésüs et al. (2007) Adiponectin is a novel humoral vasodilator? Cardiovascular Research 75 719-727.
Galano A, Cruz-Torres A. OH radical reactions with phenylalanine in free and peptide forms. Org Biomol Chem. 2008 Feb 21;6(4):732-8.
Griendling KK, Sorescu D, Ushio-Fukai M.: NAD(P)H oxidase: role in cardiovascular biology and disease. Circ Res. 2000 Mar 17;86(5):494-501. Review.
Gurer-Orhan H, Ercal N, Mare S, Pennathur S, Orhan H, Heinecke JW. Misincorporetion of free m-tyrosine into cellular proteins: a potential cytotoxic mechanism for oxidized amino acids. Biochem J. 2006 Apr 15;395(2):277-84.
Hao S, Avraham Y, Bonne O, Berry EM: Separation-induced body weight loss, impairment in alternation behavior, and autonomic tone: effects of tyrosine. Pharmacol Biochem Behav. 2001 Feb;68(2):273-81.
Hein TW, Singh U, Vasquez-Vivar J, Devaraj S, Kuo L, Jialal I.: Human C-reactive protein induces endothelial dysfunction and uncoupling of eNOS in vivo. Atherosclerosis. 2009 Sep;206(1):61-8. Epub 2009 Feb 12. Houstis N, Rosen ED, Lander ES: Reactive oxygen species have a causal role in multiple forms of insulin resistance. Nature. 2006 Apr 13;440(7086):944-8.
Huang X, Zhang J, Liu J, Sun L, Zhao H, Lu Y, Wang J, Li J.: C-reactive protein promotes adhesion of monocytes to endothelial cells via NADPH oxidase-mediated oxidative stress. J Cell Biochem. 2012 Mar;113(3):857-67. doi: 10.1002/jcb.23415.
Huggins TG, Wells-Knecht MC, Detorie NA, Baynes JW, Thorpe SR. Formation of o-tyrosine and dityrosine in proteins during radiolytic and metal-catalyzed oxidation. J Biol Chem. 1993 Jun 15;268(17):12341-7.
Iozzo P: Viewpoints on the way to the consensus session: where does insulin resistance start? The adipose tissue. Diabetes Care. 2009 Nov;32 Suppl 2:S168-73.
Kaddai V, Gonzalez T, Keslair F, Grémeaux T, Bonnafous S, Gugenheim J, Tran A, Gual P, Le Marchand-Brustel Y, Cormont M. Rab4b is a small GTPase involved in the control of the glucose transporter GLUT4 localization in adipocyte. 2009; PLoS One 4: e5257.
Kanety H, Feinstein R, Papa MZ, Hemi R, Karasik A (1995) Tumor necrosis factor alpha-induced phosphorylation of insulin receptor substrate-1 (IRS-1). Possible mechanism for suppression of insulin-stimulated tyrosine phosphorylation of IRS-1. J Biol Chem 270: 23780-23784
Kuhad A, Bishnoi M, Tiwari V, Chopra K.: Suppression of NF-kappabeta signaling pathway by tocotrienol can prevent diabetes associated cognitive deficits. Pharmacol Biochem Behav. 2009 Apr;92(2):251-9. Epub 2008 Dec 24.
Kuhad A, Chopra K.: Attenuation of diabetic nephropathy by tocotrienol: involvement of NFkB signaling pathway. Life Sci. 2009 Feb 27;84(9-10):296-301. Epub 2008 Dec 30.
Kuhad A, Chopra K.: Tocotrienol attenuates oxidative-nitrosative stress and inflammatory cascade in experimental model of diabetic neuropathy. Neuropharmacology. 2009 Sep;57(4):456-62. Epub 2009 Jun 23.
Li JM, Shah AM. ROS generation by nonphagocytic NADPH oxidase: potential relevance in diabetic nephropathy. J Am Soc Nephrol. 2003 Aug;14(8 Suppl 3):S221-6.
Mahadev K, Wu X, Zilbering A, Zhu L, Lawrence JT, Goldstein BJ: Hydrogen peroxide generated during cellular insulin stimulation is integral to activation of the distal insulin signaling cascade in 3T3-L1 adipocytes. J Biol Chem. 2001 Dec 28;276(52):48662-9.
Martinez-Hervas S, Real JT, Ivorra C, Priego A, Chaves FJ, Pallardo FV, Viña JR, Redon J, Carmena R, Ascaso JF.: Increased plasma xanthine oxidase activity is related to nuclear factor kappa beta activation and inflammatory markers in familial combined hyperlipidemia. Nutr Metab Cardiovasc Dis. 2010 Dec;20(10):734-9. Epub 2009 Sep 17.
Møller SE, Maach-Møller B, Olesen M, Madsen B, Madsen P, Fjalland B: Tyrosine metabolism in users of oral contraceptives. Life Sci. 1995;56(9):687-95.
Molnár GA, Nemes V, Biró Z, Ludány A, Wagner Z, Wittmann I: Accumulation of the hydroxyl free radical markers meta-, ortho-tyrosine and DOPA in cataractous lenses is accompanied by a lower protein and phenylalanine content of the water-soluble phase. Free Radic Res. 2005 Dec;39(12):1359-66.
Molnár GA, Wagner Z, Markó L, K6 Szegi T, Mohás M, Kocsis B, Matus Z, Wagner L, Tamaskó M, Mazák I, Laczy B, Nagy J, Wittmann I: Urinary ortho-tyrosine excretion in diabetes mellitus and renal failure: evidence for hydroxyl radical production. Kidney Int. 2005 Nov;68(5):2281-7.
Nagaoka T, Kuo L, Ren Y, Yoshida A, Hein TW.: C-reactive protein inhibits endothelium-dependent nitric oxide-mediated dilation of retinal arterioles via enhanced superoxide production. Invest Ophthalmol Vis Sci. 2008 May;49(5):2053-60.
Naito Y et al. (2010) Oxidative Stress Markers. Anti-Aging Medicine 7 (5) : 36-44.
Ogino K and Wang D-H. (2007) Biomarkers of Oxidative/Nitrosative Stress:An Approach to Disease Prevention. Acta Med. Okayama, 61(4) 181-189
Pokomy 1 et al. Antioxidants in food: practical applications. Woodhead Publishing Ltd. Cambridge, U.K. 2001 Reaven GM: Banting lecture 1988. Role of insulin resistance in human disease. Diabetes. 1988 Dec;37(12):1595-607.
Reinstein DK, Lehnert H, Wurtman RJ: Dietary tyrosine suppresses the rise in plasma corticosterone following acute stress in rats. Life Sci. 1985 Dec 9;37(23):2157-63.
Rodgers KJ, Wang H, Fu S, Dean RT: Biosynthetic incorporation of oxidized amino acids into proteins and their cellular proteolysis. Free Radic Biol Med. 2002 Apr 15;32(8):766-75.
Rudich A, Kozlovsky N, Potashnik R, Bashan N: Oxidant stress reduces insulin responsiveness in 3T3-L1 adipocytes. Am J Physiol. 1997 May;272(5 Pt 1):E935-40.
Ryan EA, Imes S, Wallace C: Short-term intensive insulin therapy in newly diagnosed type 2 diabetes. Diabetes Care. 2004 May;27(5): 1028-32.
Singh U, Devaraj S, Vasquez-Vivar J, Jialal I.: C-reactive protein decreases endothelial nitric oxide synthase activity via uncoupling. J Mol Cell Cardiol. 2007 Dec;43(6):780-91. Epub 2007 Aug 31.
Stephens J W. et al. (2009) The biological relevance and measurement of plasma markers of oxidative stress in diabetes and cardiovascular disease. Atherosclerosis 202 321-329
Thomson MJ, Williams MG, Frost SC: Development of insulin resistance in 3T3-LI adipocytes. J Biol Chem. 1997 Mar 21;272(12):7759-64.
Van der Putten K, Braam B, Jie KE, Gaillard CA (2008) Mechanisms of Disease: erythropoietin resistance in patients with both heart and kidney failure. Nat Clin Pract Nephrol. 4(1) 47-57.
Wu X, Zhu L, Zilbering A, Mahadev K, Motoshima H, Yao J, Goldstein BJ. Hyperglycemia Potentiates H2O2 Production in Adipocytes and Enhances Insulin Signal Transduction: Potential Role for Oxidative Inhibition of Thiol-Sensitive Protein-Tyrosine Phosphatases. Antioxid Redox Signal. 2005; 7(5-6): 526-537.
Yasunari K et al. (2002) Oxidative Stress in Leukocytes Is a Possible Link Between Blood Pressure, Blood Glucose, and C-Reacting Protein. Hypertension 39:777-780.
Yoshioka K, Yoshida T, Yoshikawa T: Short-term intensive insulin therapy in newly diagnosed type 2 diabetes: response to Ryan, Imes, and Wallace. Diabetes Care. 2004 Sep;27(9):2281-2
Yuan H, Zhang X, Huang X, Lu Y, Tang W, Man Y, Wang S, Xi J, Li J: NADPH oxidase 2-derived reactive oxygen species mediate FFAs-induced dysfunction and apoptosis of β-cells via JNK, p38 MAPK and p53 pathways. PLoS One. 2010 Dec 29;5(12):e15726.
Zecchin H. G. et al. (2007) Defective Insulin and Acetylcholine Induction of Endothelial Cell-Nitric Oxide Synthase Through Insulin Receptor Substrate/Akt Signaling Pathway in Aorta of Obese Rats. Diabetes 56(4) 1014-1024.

## Claims

1. A compound selected from para-L-Tyrosine (p-Tyr) and p-OH-phenyl-pyruvate, or pharmaceutically acceptable salts thereof, for use in a subject in the prevention, amelioration or treatment of a condition or disorder due to hormone resistance induced by oxidative stress, wherein said p-OH-phenyl-pyruvate is metabolized to p-Tyr in said subject.

2. The compound for use of claim 1 wherein the subject is a subject in a chronic oxidative stress disease.

3. The compound for use of claim 1 or 2, wherein the oxidative stress is **characterized by**
- an increased level of meta-Tyrosine (m-Tyr) and/or ortho-Tyrosine (o-Tyr) in a body fluid of said subject, wherein the body fluid is selected from serum, plasma, urine, saliva, liquor, tear, or in a tissue of said subject wherein the tissue is selected from adipose tissue, muscle, biopsy specimens from kidney or liver.

4. The compound for use of any of claims 1 to 3 wherein the hormone resistance is different from leptin resistance.

5. The compound for use of any of claims 1 to 4 wherein said compound is for use in the prevention of a condition or disorder due to hormone resistance induced by oxidative stress and is to be administered before the onset of said condition or disorder, wherein preferably the subject is exposed to oxidative stress or predisposed to said condition or disorder.

6. The compound for use of any of claims 1 to 5, wherein said hormone resistance is insulin resistance.

7. The compound for use of any of claims 1 to 5, wherein said hormone resistance is erythropoietin resistance.

8. The compound for use of any of claims 1 to 5, wherein said hormone resistance is acetylcholine resistance.

9. The compound for use of any of claims 1 to 8, wherein said condition is selected from hypertension, oxidative stress in adipose tissue or in fat cells, anemia, chronic anemia, ischemia, vascular diseases, chronic renal disease (CRF), chronic heart failure (CHF), oxidative stress of the kidney, obstructive sleep apnea syndrome, malignancies related to hormone resistance or any condition being a consequence thereof.

10. The compound for use of any of claims 1-9 wherein the hormone resistance is induced by oxidative stress via the formation of o-Tyr and/or m-Tyr and via incorporation of said o-Tyr and/or m-Tyr into cellular proteins, preferably into at least one cellular protein of the intracellular signalling pathway of a hormone the resistance of which said condition is related to.

11. The compound for use according to any of claims 1-9 wherein the hormone acts via the phosphatidylinositol 3-kinase(PI3K)/AKT pathway.

12. The compound for use of claim 10 wherein said at least one protein is selected from the intracellular part of a receptor of said hormone, insulin receptor IRS1, IRS2, PD3K, PDK1, Akt and GLUT4.

13. The compound for use of any of claims 1 to 12 wherein the prevention, amelioration or treatment of said condition comprises a personalized regimen tailored to said subject wherein the administration of said compound to the subject is continued for at least 7, 8, 9, 10, 11, 12, 13, 14 or 15 days, more preferably for at least one month, at least 2 months, at least 3 months, at least 4 months or at least for half year or at least 1 year or at least 2 years or at least 3 years or at least 5 years or at least 10 years or lifelong.

14. A formulation comprising a compound as defined in any of claims 1 to 13, for use in a subject in the prevention, amelioration or treatment of a condition due to hormone resistance induced by oxidative stress,
said formulation being selected from a pharmaceutical composition and a medicament.

15. A formulation comprising a compound as defined in any of claims 1 to 13, for use in a subject in the prevention, amelioration or treatment of a condition due to hormone resistance induced by oxidative stress,
said formulation being selected from a dietary supplement, nutraceutical, functional food, food and a composition with a health claim.

## Patentansprüche

1. Verbindimg ausgewählt von para-L-Tyrosin (p-Tyr) und p-Hydroxyphenylbrenztraubensäure oder ihren pharmazeutisch akzeptierbaren Salzen zur Anwendung bei einem Subjekt in der Prävention, Lindenmg oder Behandlung von auf durch oxidativen Stress verursachter Hormonresistenz beruhenden Zuständen oder Störungen, wobei die p-Hydroxyphenylbrenztraubensäure im Subjekt in p-Tyr metabolisiert wird.

2. Verbindung zur Anwendung gemäß Anspruch 1, wobei das Subjekt an einer chronischen oxidativen Stresserkrankung leidet.

3. Verbindimg zur Anwendung gemäß Anspruch 1 oder 2, wobei der oxidative Stress **gekennzeichnet ist durch**
- einen erhöhten Spiegel an meta-Tyrosin (m-Tyr) und/oder ortho-Tyrosin (o-Tyr) in einer Körperflüssigkeit des Subjekts, wobei die Körperflüssigkeit aus Semm, Plasma, Urin, Speichel, Liquor, Tränenflüssigkeit ausgewählt ist, oder in einem Gewebe des Subjekts, wobei das Gewebe aus Fettgewebe, Muskel, eine Biopsieprobe aus Niere oder Leber ausgewählt ist.

4. Verbindung zur Anwendung gemäß einem der Ansprüche 1-3, wobei die Hormonresistenz keine Leptinresistenz ist.

5. Verbindung zur Anwendung gemäß einem der Ansprüche 1-3, wobei die Verbindung der Anwendung bei Subjekten in der Prävention, Linderung oder Behandlung von auf durch oxidativen Stress verursachter Hormonresistenz bemhenden Zuständen oder Stönmgen dient und vor Beginn des Zustandes oder der Störung zu verabreichen ist, wobei das Subjekt insbesondere oxidativem Stress ausgesetzt ist oder anfällig gegenüber dem Zustand oder der Störung ist.

6. Verbindimg zur Anwendimg gemäß einem der Ansprüche 1-5, wobei die Hormonresistenz eine Insulinresistenz ist.

7. Verbindimg zur Anwendung gemäß einem der Ansprüche 1-5, wobei die Hormonresistenz eine Erythropoietinresistenz ist.

8. Verbindimg zur Anwendung gemäß einem der Ansprüche 1-5, wobei die Hormonresistenz eine Acetylcholinresistenz ist.

9. Verbindung zur Anwendung gemäß einem der Ansprüche 1-8, wobei der Zustand ausgewählt ist aus Hypertonie, oxidativem Stress in Fettgewebe oder Fettzellen, Anämie, chronischer Anämie, Ischämie, Gefäßerkrankungen, chronischer Niereninsuffizienz (CRF), chronischer Herzinsuffizienz (CHF), oxidativem Stress der Niere, obstmktivem Schlafapnoe-Syndrom, mit Hormonresistenz zusammenhängenden bösartigen Tumoren oder daraus resultierenden Zuständen.

10. Verbindimg zur Anwendimg gemäß einem der Ansprüche 1-9, wobei die Hormonresistenz durch oxidativen Stress durch Bildung von o-Tyr und/oder m-Tyr und Aufnahme des o-Tyr und/oder m-Tyr in zelluläre Proteine, vorzugsweise in mindestens ein zelluläres Protein des intrazellulären Signalweges des Hormons, auf dessen Resistenz der Zustand zurückgeführt wird, verursacht wird.

11. Verbindung zur Anwendung gemäß einem der Ansprüche 1-9, wobei das Hormon über den Phosphatidylinositol-3-Kinase (PI3K)/AKT-Weg wirkt.

12. Verbindung zur Anwendung gemäß Anspruch 10, wobei das mindestens ein Protein ausgewählt ist aus dem intrazellulären Teil eines Rezeptors des Hormons, aus dem Insulinrezeptor IRS1, IRS2, PD3K, PDK1, Akt und GLUT4.

13. Verbindung zur Anwendung gemäß einem der Ansprüche 1-12, wobei die Prävention, Lindenmg oder Behandlung des Zustandes ein individuell auf das betreffende Subjekt zugeschnittenes Behandlungsregime beinhaltet, wobei die Verabreichung der Verbindung an das Subjekt mindestens 7, 8, 9, 10, 11, 12, 13, 14 oder 15 Tage lang, vorteilhafter mindestens einen Monat, mindestens zwei Monate, mindestens drei Monate, mindestens vier Monate lang oder mindestens ein halbes Jahr lang oder mindestens ein Jahr lang oder mindestens zwei Jahre lang oder mindestens 3 Jahre lang oder mindestens 5 Jahre lang oder mindestens 10 Jahre lang oder lebenslang erfolgt.

14. Zubereitung enthaltend eine Verbindung gemäß einem der Ansprüche 1-13 zur Anwendung bei Subjekten in der Prävention, der Linderung oder Behandlung eines auf durch oxidativen Stress verursachter Hormonresistenz beruhenden Zustandes, die ausgewählt ist aus einer pharmazeutischen Zusammensetzung oder einem Medikament.

15. Zubereitung enthaltend eine Verbindung gemäß einem der Ansprüche 1-13 zur Anwendung bei Subjekten in der Prävention, der Linderung oder Behandlung eines auf durch oxidativen Stress verursachter Hormonresistenz beruhenden Zustandes, die ausgewählt ist aus einem diätetischen Zusatz, Nutrazeutikum, funktionellen Lebensmittel, Lebensmittel und einer Zusammensetzung mit nährwert- und gesundheitsbezogenen Angaben.

## Revendications

1. Un composé sélectionné parmi la para-L-Tyrosine (p-Tyr) et le p-OH-phényle-pyruvate, ou un sel pharmaceutiquement acceptable de ceux-ci, pour son utilisation dans un sujet dans la prévention, l'amélioration ou le traitement d'une maladie ou d'un trouble causé par une résistance hormonale induite par un stress oxydatif, où ledit p-OH-phényle-pyruvate est métabolisé en p-Tyr dans ledit sujet.

2. Le composé pour son utilisation selon la revendication 1 où le sujet est un sujet souffrant d'une maladie du stress oxydatif chronique.

3. Le composé pour son utilisation selon les revendications 1 ou 2, où le stress oxydatif est caractérisé en
- un taux élevé de la méta-Tyrosine (m-Tyr) et/ou de l'ortho-Tyrosine (o-Tyr) dans un liquide corporel dudit sujet, où le liquide corporel est sélectionné parmi le sérum, le plasma, l'urine, la salive, la liqueur, les larmes, ou dans un tissu dudit sujet où le tissu est sélectionné parmi le tissu adipeux, le muscle, les prélèvements biopsiques de la rein ou du foie.

4. Le composé pour son utilisation selon l'une quelconque des revendications 1 à 3 où la résistance hormonale est différente de la résistance à la leptine.

5. Le composé pour son utilisation selon l'une quelconque des revendications 1 à 4 où ledit composé est à utiliser dans la prévention d'une maladie ou d'un trouble causé par une résistance hormonale induite par un stress oxydatif et doit être administré avant du début de ladite maladie ou dudit trouble, où préférablement le sujet est exposé au stress oxydatif ou prédisposé à ladite maladie ou ledit trouble.

6. Le composé pour son utilisation selon l'une quelconque des revendications 1 à 5, où ladite résistance hormonale est la résistance à l'insuline.

7. Le composé pour son utilisation selon l'une quelconque des revendications 1 à 5, où ladite résistance hormonale est la résistance à l'érythropoïétine.

8. Le composé pour son utilisation selon l'une quelconque des revendications 1 à 5, où ladite résistance hormonale est la résistance à l'acétylcholine.

9. Le composé pour son utilisation selon l'une quelconque des revendications 1 à 8, où ladite maladie est sélectionnée parmi l'hypertension, le stress oxydatif dans un tissu adipeux ou dans les cellules grasses, l'anémie, l'anémie chronique, l'ischémie, les maladies vasculaires, la maladie rénale chronique (IRC), l'insuffisance cardiaque chronique (ICC), le stress oxydatif de la rein, le syndrome de l'apnée obstructive du sommeil, les malignités relatives à une résistance hormonale ou une quelconque maladie étant une conséquence de celle-ci.

10. Le composé pour son utilisation selon l'une quelconque des revendications 1-9 où la résistance hormonale est induite par un stress oxydatif via la formation de l'o-Tyr et/ou de la m-Tyr et via l'incorporation de ladite o-Tyr et/ou de ladite m-Tyr dans les protéines cellulaires, préférablement dans au moins une protéine cellulaire de la voie de signalisation intracellulaire d'une hormone la résistance à laquelle ladite maladie est liée.

11. Le composé pour son utilisation selon l'une quelconque des revendications 1-9 où la hormone agit via la voie de la phosphatidylinositol 3-kinase(PI3K)/AKT.

12. Le composé pour son utilisation selon la revendication 10 où ladite au moins une protéine est sélectionnée parmi la partie intracellulaire d'un récepteur de ladite hormone, un récepteur d'insuline IRS1, IRS2, PD3K, PDK1, Akt et GLUT4.

13. Le composé pour son utilisation selon l'une quelconque des revendications 1 à 12 où la prévention, l'amélioration ou le traitement de ladite maladie comprend un régime personnalisé adapté audit sujet où l'administration dudit composé au sujet est continuée pendant au moins 7, 8, 9, 10, 11, 12, 13, 14 ou 15 jours, plus préférablement pendant au moins un mois, au moins 2 mois, au moins 3 mois, au moins 4 mois, ou au moins pendant une demi année, ou au moins 1 année, ou au moins 2 ans, ou au moins 3 ans, ou au moins 5 ans, ou au moins 10 ans, ou pendant toute une vie.

14. Une formulation comprenant un composé défini dans l'une quelconque des revendications 1 à 13, pour son utilisation dans un sujet pour la prévention, l'amélioration ou le traitement d'une maladie causée par une résistance hormonale induite par un stress oxydatif,
ladite formulation étant sélectionnée parmi une composition pharmaceutique et un médicament.

15. Une formulation comprenant un composé défini dans l'une quelconque des revendications 1 à 13, pour son utilisation dans un sujet pour la prévention, l'amélioration ou le traitement d'une maladie causée par une résistance hormonale induite par un stress oxydatif,
ladite formulation étant sélectionnée parmi un complément alimentaire, une nutracéutique, un aliment fonctionnel, un aliment et une composition avec une allégation de santé.
